# EUROPÄISCHE PATENTANMELDUNG

(11) **EP 0 637 631 A2**
(43) Veröffentlichungstag der Anmeldung: **08.02.1995**
(21) Anmeldenummer: 94111872.1
(22) Anmeldetag: 29.07.1994
(51) Int. Cl.: C12N 15/86, C12N 15/51, C12N 7/01, C12P 21/00, C07K 14/02, A61K 39/29, C12P 21/08, G01N 33/576

(54) **Hoch effiziente Expression eines Polypeptids, das eine modifizierte preS1-Region des grossen Hepatitis B-Virus-Antigens enthält**

(30) Priorität: 30.07.1993 US 99351
(71) Anmelder: IMMUNO Aktiengesellschaft, A-1221 Wien (AT)
(72) Erfinder: Dorner, Friedrich, Prof., A-1230 Wien (AT); Pfleiderer, Michael, Dr., A-2285 Breitenstetten 19 (AT); Falkner, Falko-Günter Dr.,, A-2304 Mannsdorf 116 (AT)
(74) Vertreter: Kolb, Helga, Dr. Dipl.-Chem.

(57) **Zusammenfassung**

Die vorliegende Erfindung betrifft Verfahren zur Herstellung rekombinanter Moleküle, die die Nucleotidsequenz für die Struktur der preS1-Region des Oberflächenglycoproteins des Hepatitis B-Virus umfassen, und Zusammensetzungen aus diesen Molekülen. Die Zusammensetzungen aus den rekombinanten Molekülen können einen Promotor und ein Markergen umfassen. Klonierungsvektoren werden verwendet, um die rekombinanten Moleküle in Wirtsvektoren zu integrieren (Vaccinia Virus). Mit den chimären Vaccinia-Viren infizierte Zellen bilden große Mengen an preS1-Protein. Isolierung und Reinigung des preS1 enthaltenden Proteins werden durch die Verwendung eines rekombinanten Moleküls erleichtert, bei dem die Myristylierungsstelle durch Modifikation der Nucleotidsequenz deletiert worden ist. Das gereinigte, preS1 enthaltenden Protein eignet sich für die Entwicklung von Impfstoffen, diagnostischen Reagenzsätzen und Therapien.

## Beschreibung

Die Verhinderung und Behandlung viraler Infektionen sind ein wesentliches medizinisches Ziel. Erkrankungen, die durch nicht-passagierbare Viren (NP-Viren) hervorgerufen werden, sind besonders schwer zu behandeln, da die sie hervorrufenden Agenzien nicht ohne weiteres in Laboratorien für die Untersuchung der die Krankheit hervorrufenden Mechanismen oder für die Entwicklung von Impfstoffen oder Therapien gezüchtet werden können. Die Erkrankungen, die durch NP-Viren hervorgerufen werden, sind oftmals sehr ernsthaft, unter Einschluß von Krebserkrankungen, Störungen des Immunsystems und Entkräftung.

Das Hepatitis B-Virus oder HBV ist ein für den Menschen hochgradig infektiöses NP-Virus. In vielen afrikanischen und asiatischen Ländern ist die HBV-Infektion endemisch. Große Teile dieser Populationen sind Träger des Virus, und daher ist eine Ausbreitung der Erkrankung überaus schwer zu bekämpfen. In den entwickelteren Ländern sind Bluttransfusionen eine hauptsächliche Übertragungsart.

HBV enthält drei Oberflächenglycoproteine unterschiedlicher Größe, die von einem einzelnen offenen Leserahmen codiert werden. Bei jedem wird ein anderes ATG-Startcodon verwendet, alle drei nutzen jedoch das gleiche TAA-Stopsignal; Ganem & Varmus, 1987; Robinson, 1990. Bei dem HBV-Stamm ayw enthält das kleine Oberflächenantigen S-sAg (Hauptantigen bzw. Hauptprotein) 226 Aminosäuren. Das mittlere Oberflächenglycoprotein preS2-S-sAg (mittleres Antigen bzw. mittleres Protein) weist zusätzliche 55 Aminosäuren am Aminoterminus von S-sAg auf. Entsprechend weist das große Oberflächenglycoprotein preS1-S2-S-sAg (großes Antigen bzw. großes Protein) weitere 108 Aminosäuren auf, die mit dem Aminoterminus von preS2-S-sAg verknüpft sind. Die nachstehende Verwendung der Ausdrücke "preS1"-Region oder "preS2"-Region beziehen sich auf die Segmente mit den zusätzlichen 108 bzw. 55 Aminosäuren, die für das große bzw. das mittlere Antigen gefunden werden.

Das große Protein ist an seinem N-terminalen Glycinrest myristyliert; Persing et al., 1987. Das Molekulargewicht der einzelnen Antigene hängt von der Glycosylierung ab und beträgt 24 oder 27 kD für das kleine Protein, 30 oder 33 kD für das mittlere Protein und 39 oder 42 kD für das große Protein; Ganem & Varmus, 1987.

Die preS-Regionen binden an Rezeptoren auf der Oberfläche von Leberzellen entweder über polymerisiertes menschliches Serumalbumin (preS2-Region) oder durch direkte Bindung der preS1-Region an das Rezeptormolekül; Pontisso et al., 1989a; Neurath et al., 1990; Pontisso et al., 1989b; Petit et al., 1992. PreS1-S2-S-sAg bleibt mit dem endoplasmatischen Reticulum (ER) assoziiert und verläßt die infizierte Zelle nur als Komponente eines infektiösen Viruspartikels (22 nm) oder als Komponente von filamentösen, nicht-infektiösen Partikeln (42 nm) in der späten Infektionsphase; Hollinger, 1990. Im Gegensatz dazu werden das mittlere Antigen und das Hauptantigen effizient sezerniert und assoziieren zu nicht-infektiösen 22nm Partikeln. Mechanismen, die die Translokation des mittleren Antigens und des Hauptantigens durch die Membran des ER vermitteln oder die Sekretion des großen Antigens verhindern, sind beschrieben worden; Ou & Rutter, 1987; Masuda et al., 1990; Prange et al., 1992.

Alle drei HBV-Oberflächenproteine induzieren eine B-Zell- und T-Zell-spezifische Immunantwort bei infizierten Individuen. In ungefähr 90 % der Fälle führen diese Immun-Antworten zu einer vollständigen Clearance des infektiösen Virus; Hollinger, 1990. Bei einer ausführlichen Epitop-Analyse der Oberflächenglycoproteine sind diskrete B-Zell- und T-Zell-spezifische neutralisierende Epitope sowohl beim Hauptantigen als auch in den preS-Regionen gefunden worden; Neurath et al., 1985; Milich et al., 1985a; Milich et al., 1985b; Neurath et al., 1989.

Das Hepatitis B-Virus-Antigen besteht aus einem gruppenspezifischen Antigen a und mindestens zwei Subtyp-Determinanten d/y und w/r (Le Bouvier, 1971; Bancroft et al., 1972), was zu mehreren unterschiedlichen antigenen genomischen Subtypen führt: adw, ayw, adr und ayr. Die geographische Verteilung der Subtypen ist von Nishioka et al. (1975) beschrieben worden. Eine Subtyp übergreifende Immunantwort ist von Szmuness et al. (1982) gezeigt worden. Einige der Subtyp-spezifischen Determinanten befinden sich in den pres-Regionen (z.B. Milich et al., 1990a & 1990b).

Impfstudien mit unterschiedlichen Mäusestämmen haben gezeigt, daß die Antwort auf bestimmte S-sAg-Epitope vom Vorhandensein definierter Moleküle im Haupthistokompatibilitätskomplex abhängt. Bestimmte Stämme, die nicht auf das Hauptprotein ansprechen, sprechen jedoch auf zusätzliche Epitope in den preS-Regionen des großen Proteins an. Das fehlende Ansprechen von Mäusen auf Impfstoffe, die auf dem Hauptantigen allein basieren, kann daher umgangen werden, indem das große Protein eine Komponente des Impfstoffes darstellt; Milich et al., 1985a & 1985b.

Bei Menschen variiert die Immunantwort von nicht ansprechend über gering ansprechend zu stark ansprechend. Diese Wirkung scheint Haplotypabhängig zu sein; Egea et al., 1991. Während Impfstoffe, die auf dem Hauptantigen allein basieren, in den meisten Fällen ausreichen, um eine Antikörperantwort beim Menschen hervorzurufen, die zu einem Schutz vor der Krankheit führt (Szmuness et al., 1980), spricht ein bestimmter Prozentsatz der Impfkandidaten nicht auf Impfstoffe an, die auf dem S-sAg alleine basieren. In einer Studie sprachen 7 bis 15 % derjenigen, die mit S-sAg geimpft wurden, nicht durch eine Bildung von Antikörpern an; Nowicki et al., J. Infect. Dis., Bd. 152 (1985), 1245-1258. Impfstoffe, die teilweise, hauptsächlich oder ausschließlich auf dem großen Antigen basieren, sind daher geeignet für die Impfung von Patienten, die schlecht auf Impfstoffe ansprechen, die nur das Hauptantigen enthalten.

In einigen Berichten ist festgestellt worden, daß Kombinationen von Antigenen eine Verbesserung der Ansprechraten zeigen, was darauf hinweist, daß ein Kandidat-Impfstoff, um möglichst wirksam zu sein, die antigenen Komponenten aller drei Oberflächenglycoproteine enthalten sollte. Um die optimalen Kombinationen von Antigenen und die optimalen Verhältnisse dieser Antigene zu bestimmen, ist es erforderlich verschiedene antigene Komponenten von HBV zu isolieren und zu reinigen um ausreichende Mengen zur Durchführung geeigneter Untersuchungen zur Verfügung zu haben.

Ungeachtet intensiver Anstrengungen in dieser Hinsicht bleibt die antigene Struktur und Funktion verschiedener HBV-Antigene weitgehend unbekannt. Eines der Hindernisse bei der Gewinnung von Informationen über HBV-Antigene ist die Schwierigkeit, ausreichende Mengen dieser Polypeptide zu erhalten. Ein weiteres Problem ist die Isolierung einzelner HBV-Komponenten für die Entwicklung von Impfstoffen und Arzneistoffen mit allgemeiner Wirksamkeit gegen eine Reihe von infektiösen HBV-Stämmen.

Einige antigene Komponenten können aus dem Serum oder aus Geweben infizierter Menschen oder anderer Primaten isoliert werden. Diese Strategie führt jedoch zu inakzeptabel kleinen Mengen. Bei Antigenen, die aus infizierten Zellen, Blut oder gereinigten Viren hergestellt werden, besteht auch das Risiko einer Verunreinigung mit dem aktiven Virus. Daher erscheint eine Isolierung und Reinigung der antigenen Komponenten von HBV in geeigneten Mengen nur durch rekombinante Techniken akzeptabel zu sein. Es ist zu erwarten, daß größere Mengen und reinere Proben an antigenen Komponenten im Vergleich zu denjenigen, die aus Geweben infizierter Individuen erhältlich sind, mit rekombinanten Methoden erhalten werden können. Die Herstellung von Impfstoffen und die Entwicklung von Therapien sollte also durch die verbesserten Möglichkeiten der Herstellung und Reinigung, die die in vitro Expression von rekombinanten HBV-Proteinen bietet, unterstützt werden.

Die Expression des kleinen, mittleren und großen HBV-Oberflächenglycoproteins in verschiedenen prokaryontischen und eukaryontischen Expressionssystemen ist bereits früher demonstriert worden; Yu et al., 1990; Lin et al., 1991, Schödel et al., 1991; Kumar et al., 1992. Diese Systeme umfassen Hefe (Dehoux et al., 1986; Imamura et al., 1987; Shiosaki et al., 1991; Kuroda et al., 1992), Säugerzellen (Ou & Rutter, 1987; Lee et al., 1989; Youn et al., 1989; Korec et al., 1992) und virale Expressionssysteme (McLachlan et al, 1987; Yuasa et al., 1991; Shiraki et al., 1991; Belyaev et al., 1991). Hefeexpressionssysteme exprimieren jedoch überglycosylierte große HBV-Antigene und nicht-glycosylierte Hauptproteine, wobei keines dieser Proteine sezerniert wird. Prokaryontische Systeme glycosylieren Polypeptide nicht, und sie sezernieren auch keine rekombinanten Proteine ab.

Das bereits publizierte System für die großtechnische Expression von HBV-Oberflächenglycoproteinen für die Herstellung von Impfstoffen beinhaltet ein Hefeexpressionssystem, das konstitutiv diese Proteine exprimiert. Transformierte Hefezellen bilden das kleine HBV-Oberflächenglycoprotein mit Charakteristika, die den Partikeln von 22 nm entsprechen, die im Blut von natürlichen infizierten Personen gefunden werden. Das Hauptprotein, das in Hefe gebildet wird, ist jedoch nicht glycosyliert (vgl. EP 0 414 374 A2, Seite 16), während das große Antigen überglycosyliert ist. Um diesen Nachteil zu überwinden, sind große Antigene mit deletierten Glycosylierungsstellen konstruiert worden (EP 0 414 374 A2, Beispiel F.3). Es ist jedoch wahrscheinlich, daß derartige Modifikationen die natürliche Immunogenität des Antigens verringern.

Bei natürlichen Infektionen mit HBV wird das große Oberflächenglycoprotein von einer preS1-spezifischen mRNA translatiert, während das mittlere und das kleine Protein von einer preS2-spezifischen mRNA translatiert werden (Robinson, 1990). Die transkriptionalen Promotorelemente für das preS1-S2-S-Gen befinden sich stromaufwärts von diesem Gen, während das preS2-S-Gen seine eigenen Promotorelemente aufweist, die sich innerhalb der preS1-Region befinden. In transformierten Zellinien, die ein integriertes preS1-S2-S-Gen enthalten, werden sowohl die 2,4 kb große preS1-S2-S-mRNA als auch die 2,1 kb große preS2-S-mRNA transkribiert, und alle drei Antigene werden exprimiert. Permanente Zellinien, die das preS1-S2-S-Gen integriert haben, sezernieren Partikel, die hauptsächlich aus dem Haupt-S-Antigen bei variierenden Mengen an M- und L-Antigen bestehen (WO 90/10058). In eukaryontischen Zellen exprimierte HBV-Oberflächenglycoproteine werden wirksamer sezerniert, die Ausbeute an rekombinantem Protein ist jedoch geringer als in Prokaryonten oder Hefen. Bis jetzt ist nur in Chinese Hamster Ovary cells (CHO-Zellen) eine kontinuierliche Expression rekombinanter HBV-Oberflächenglycoprotein-Gene gezeigt worden; Ou & Rutter, 1987; Lee et al., 1989; Youn et al., 1989; Korec et al., 1992.

Im Vaccinia-Virus-Expressionssystem sind HBV-Oberflächenglycoproteine hauptsächlich unter der Kontrolle des schwachen Vaccinia-Promotors P7.5 exprimiert worden. Da die Expressionsausbeuten unter Verwendung dieses Promotors gering sind, konnte das große Antigen nur unter Anwendung empfindlicher Nachweismethoden, wie radioaktiver Markierung und Autoradiographie, nachgewiesen werden; Cheng et al., 1986; Cheng et al., 1987; Kutinivà et al., 1990; Nemeckovà et al., 1991. Es ist berichtet worden, daß die Expression des kleinen und mittleren Antigens im Vaccinia-Virussystem zu einer wirksamen Sekretion beider Polypeptide führt; Cheng et al., 1987. Das große Antigen, das mit Vaccinia-Virus exprimiert wird, wird jedoch nicht sezerniert, sondern bleibt assoziiert mit intrazellulären Membranen; Cheng et al., 1986.

Zur Verbesserung der Expressionsergebnisse mit Vaccinia-Virus als Expressionsvehikel haben Fuerst et al. (1987) den normalen Promotor P7.5 durch den Bakteriophagen-T7-Promotor ersetzt. Außerdem wurde das T7-RNA-Polymerase-Gen in ein Plasmid oder Vaccinia-Virus integriert, um die Transkription vom heterologen T7-Promotor zu erreichen. Die Ergebnisse zeigten, daß mit drei unterschiedlichen Proteinen, nämlich dem E. coli β-Galactosidase, dem kleinen HBV-Antigen und dem HIV gp160, die Expression von einem T7-Promotor durch eine T7-RNA-Polymerase höher war als die, die mit nativen Vaccinia-Promotoren erreicht wurde.

Elroy-Stein et al. (1989) haben noch eine weitere Variation des Vaccinia-Virus-Expressionssystems entwickelt. Unter Verwendung des bereits beschriebenen Hybrid-T7-Vaccinia-Systems haben sie zusätzlich die 5'-nichttranslatierte Region (UTR) des Encephalomyocarditis-Virus integriert, um eine Verbesserung der Translation der mRNA, die durch die T7-RNA-Polymerase transkribiert wird, zu erwirken. Die Ergebnisse zeigten eine gesteigerte Expression des Chloramphenicol-Transferase-Gens (cat-Gens) mit Proteinkonzentrationen, die 10 % des gesamten Zellproteins erreichten.

Ein interessantes Ergebnis der Expressionsklonierung des preS1-preS2-S-sAg-Gens mit einen Vaccinia-Expressionsvektor ist die Expression des großen Antigens ohne die gleichzeitige Expression des kleinen oder mittleren Antigens. Dies steht im Gegensatz zu transformierten Zellinien, bei denen alle drei Antigene vom preS1-preS2-S-sAg-Gen exprimiert werden. Die vorliegende Erfindung zeigt also, daß Vaccinia-Systeme die unabhängige Expression des großen Proteins erlauben, was den Anteil des relevanten preS1-Region-Epitops erhöht.

Im Gegensatz zu allen anderen Berichten zeigen Li et al. (US-Patent 5 077 213), daß ein rekombinantes Vaccinia-Virus (Stamm Guang), das das preS1-S2-S-Gen aus dem HBV-Stamm adr trägt, die effiziente Sekretion des kleinen, mittleren und großen Antigens aus TK-Osteosarkom-Zellen, die mit dieser Rekombinante infiziert sind, erlaubt. Es ist nicht ersichtlich aus Li et al., welche Faktoren für die beschriebenen Ergebnisse verantwortlich sind, zumal in anderen Berichten gezeigt wird, daß (i) Vaccinia kein großes Antigen sezerniert und (ii) Vaccinia nicht imstande ist, das kleine und das mittlere Antigen vom nativen HBV-Promotor zu exprimieren. Außerdem ist es schwierig, irgendwelche allgemein gültigen Prinzipien in dieser Hinsicht zu erkennen, da Li et al. neoplastische Zellen (Osteosarkom-Zellen) als Wirt verwendeten. Die ungewöhnlichen Charakteristika dieser Zellen mögen für die abweichenden Ergebnisse, die von Li et al. berichtet werden, verantwortlich sein.

Die vorliegende Erfindung beinhaltet ein Verfahren zur Herstellung eines HBV-preS1-enthaltenden Antigens unter Verwendung eines Vaccinia-Virus-Expressionsvektors. Die Deletion der Myristylierungsstelle am Aminoterminus des großen HBV-Antigens erlaubt eine wesentlich höhere Expression derart modifizierter HBV preS1-preS2-S-sAg Polypeptide. Die Expression des demyristylierten Antigens mit Vaccinia-Virus kann (i) unter Verwendung eines starken Vaccinia-Promotors oder (ii) unter Verwendung einer Bacteriophage T7 -Promotor-Sequenz in Verbindung mit der Bacteriophage T7-RNA-Polymerase erfolgen. Die Demyristylierung des rekombinanten großen HBV-Antigens führt zu Expressionsausbeuten, die nach dem Stand der Technik nicht vorhergesagt worden wären. Wahlweise kann das großen HBV-Antigen mit einem Immunopotentiator kombiniert werden. Im Fall eines Polypeptid-Immunopotentiators kann das große Antigen durch eine chemische Verknüpfung oder durch rekombinante Verfahren, d.h. Ligieren von DNA-Sequenzen, die für den Immunopotentiator und das große HBV-Antigen codieren, um ein neues Hybridgen zu bilden, gekoppelt werden. Schließlich erlaubt die Verwendung des Vaccinia-Virus-Expressionssystems die Verwendung von Zellen als Wirte, in denen die HBV-Replikation normalerweise erfolgt. Die resultierende Verfügbarkeit großer Mengen an gereinigtem großem HBV-Antigen, das in "natürlicher" Weise prozessiert wurde, ermöglicht die Etablierung eines Anwendungsbereiches, z.B. diagnostische, therapeutische und Impfanwendungen, was bisher nicht möglich war.

Eine Aufgabe der vorliegenden Erfindung besteht daher darin, eine DNA-Sequenz zur Verfügung zu stellen, die für ein preS1-enthaltendes Antigen codiert, dem die Myristylierungsstelle fehlt, die in der preS1-Region des großen HBV-Antigens vorhanden ist. Die Myristylierungsstelle kann durch eine gezielte Mutagenese der Stelle, an der die Myristylierung stattfindet, oder durch vollständige Deletion der Stelle zerstört werden. Das Konstrukt kann anschließend in das Genom eines ersten chimären Vaccinia-Virus-Vektors unter der Kontrolle einer regulatorischen Vaccinia-Sequenz cloniert werden. Nach der Infektion von Zellen mit dem Virus wird das demyristylierte Protein in großen Mengen synthetisiert.

Eine weitere Aufgabe der vorliegenden Erfindung besteht darin, einen chimären Vaccinia-Virus-Vektor bereitzustellen, der eine demyristylierte Version eines preS-enthaltenden Antigens unter der Kontrolle eines Bacteriophage-T7-Promotors exprimiert. Ein zweiter chimärer Vaccinia-Virus-Vektor codiert für die bakterielle T7-Phagen-RNA-Polymerase. Durch Coinfektion von Zellen mit dem ersten und dem zweiten Virus wird das preS1 enthaltende Antigen in großen Mengen gebildet.

Eine weitere Aufgabe der vorliegenden Erfindung besteht darin, ein preS1 enthaltendes Antigen gemäß einem der vorstehenden Ziele bereitzustellen, wobei das Antigen durch Infektion von Zellen, die aus Leber oder Niere stammen, synthetisiert wird. Das Antigen wird somit in einer Weise prozessiert, die mit seiner natürlichen Antigenität und Immunogenität übereinstimmt.

Es ist weiterhin eine Aufgabe der vorliegenden Erfindung, preS1 enthaltendes Antigen in einer gereinigten oder teilweise gereinigten Form bereitzustellen. Die Gewinnung eines derartigen Antigens wird problemlos in einem auf Vaccinia basierenden Expressionssystem erreicht, da hier der interne Promotor, der für die Synthese des Transkripts des kleinen/mittleren Antigens sorgt, nicht aktiv ist. Daher wird kein kleines oder mittleres Antigen gebildet.

Eine weitere Aufgabe der vorliegenden Erfindung besteht darin, einen preS1 enthaltenden Impfstoff gemäß den vorstehenden Zielen bereitzustellen. Die Bedeutung eines derartigen Produkts wird durch die Eigenschaft der preS1-Region gezeigt, einem bestimmten Prozentsatz von Personen eine Immunität zu verleihen, die nicht auf Standard-HBV-Impfstoffe ansprechen.

Es ist eine weitere Aufgabe der vorliegenden Erfindung, einen preS1 enthaltenden Impfstoff bereitzustellen, der auch einen Immunopotentiator enthält, der durch genetische Fusion der für preS1 und den Immunopotentiator codierenden Sequenzen oder durch chemische Kopplung erhalten wird.

Eine weitere Aufgabe der vorliegenden Erfindung besteht darin, ein preS1 enthaltendes Antigen bereitzustellen, wobei mehr als eine Kopie der preS1-Region vorhanden ist.

Indem die vorstehenden Ziele erreicht werden, wird gemäß einem Aspekt der vorliegenden Erfindung ein chimäres Vaccinia-Virus bereitgestellt, das einen Promotor umfaßt, der funktional mit einem Polynucleotid verknüpft ist, das für einen Bereich eines großen Antigens eines Hepatitis B-Virus codiert, der mindestens ein preS1-B- oder -T-Zell-Epitop enthält, wobei dem Bereich die Myristylierungsstelle der preS1-Region fehlt.

Gemäß einem weiteren Aspekt der Erfindung wird ein Verfahren zur Synthese eines Hepatitis B-Virus-Antigens bereitgestellt, das folgende Stufen umfaßt: (a) Infektion einer geeigneten eukaryontischen Zelle mit einem ersten chimären Vaccinia-Virus, das einen Promotor umfaßt, der funktional mit einem Polynucleotid verknüpft ist, das für einen Bereich der preS1-Proteinregion eines großen Hepatitis B-Virus-Antigens codiert, der mindestens ein preS1-B- oder -T-Zell-Epitop enthält, wobei dem Bereich die Myristylierungsstelle der preS1-Region fehlt; (b) Kultivieren der coinfizierten Zelle; (c) Lyse der coinfizierten Zelle; und (d) Gewinnung des Antigens.

Gemäß einer bevorzugten Ausführungsform der vorliegenden Erfindung wird ein Hepatitis B-Virus-Antigen nach einem Verfahren hergestellt, das folgende Stufen umfaßt: (a) Coinfizieren einer geeigneten eukaryontischen Zelle mit einem ersten chimären Vaccinia-Virus, das einen Promotor umfaßt, der funktional mit einem Polynucleotid verknüpft ist, das für einen Bereich der preS1-Proteinregion eines großen Hepatitis B-Virus-Antigens codiert, der mindestens ein preS1-B- oder -T-Zell-Epitop enthält, wobei dem Bereich die Myristylierungsstelle der preS1-Region fehlt; (b) Kultivieren der coinfizierten Zelle; (c) Lyse der coinfizierten Zelle; und (d) Gewinnung des Antigens.

Gemäß einer weiteren bevorzugten Ausführungsform ist das Hepatitis B-Virus-Antigen mit einem Immunopotentiator verknüpft.

Gemäß einer weiteren Ausführungsform wird ein Impfstoff bereitgestellt, der ein Antigen eines Hepatitis B-Virus, das nach dem vorstehend beschriebenen Verfahren hergestellt wurde, in einem pharmazeutisch verträglichen Träger umfaßt.

Gemäß einer weiteren Ausführungsform wird ein anti-HBV-preS1-Antikörper bereitgestellt, der durch Immunisierung eines Säugers mit einem Antigen des Hepatitis B-Virus, das nach dem vorstehend beschriebenen Verfahren hergestellt wurde, hergestellt wird.

Außerdem werden diagnostische Reagenzsätze bereitgestellt, die entweder ein Hepatitis B-Virus-Antigen oder einen anti-HBV-preS1-Antikörper umfassen.

Weitere Aufgaben, Merkmale und Vorteile der vorliegenden Erfindung sind aus der nachstehenden ausführlichen Beschreibung ersichtlich. Es ist jedoch darauf hinzuweisen, daß die ausführliche Beschreibung und die speziellen Beispiele zwar bevorzugte Ausführungsformen der Erfindung bezeichnen, jedoch nur zur Erläuterung vorgelegt werden, da verschiedene Änderungen und Modifikationen innerhalb des Wesens und des Schutzumfangs der Erfindung für den Fachmann aus der vorliegenden Offenbarung deutlich werden.

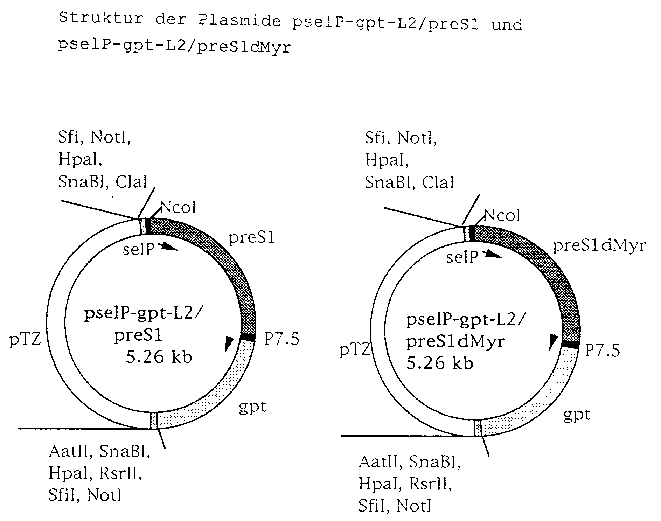

Fig. 1 - Struktur der Plasmide pselP-gpt-L2/preS1 und pselP-gpt-L2/preS1dMyr. Die Plasmidstrukturen des Gens für das große HBV-Protein bzw. das demyristylierte große HBV-Protein sind angegeben. Pfeile zeigen die Richtung der Transkription von den Promotoren selP und P7.5.

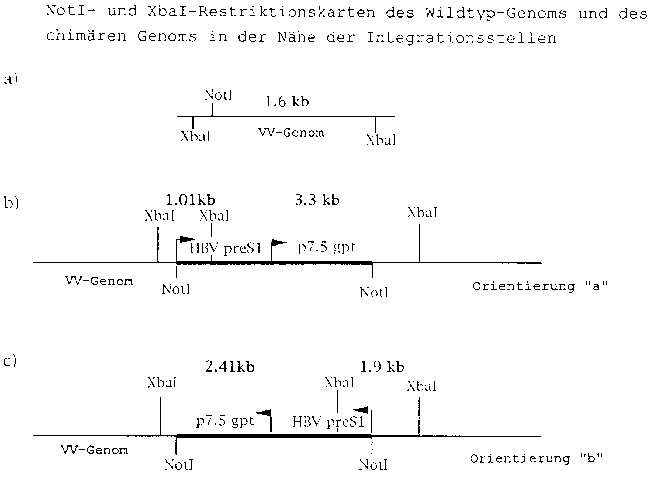

Fig. 2 - XbaI-Restriktionsmuster von chimären preS1- oder preS1dMyr-Vaccinia-Viren. Die Größen der XbaI-Fragmente der chimären Vaccinia-Viren, die mit einer ³²P-markierte, preS1-spezifische Sonde hybridisieren, sind gezeigt. Pfeile zeigen die Richtung der Transkription von den Promotoren selP und P7.5.

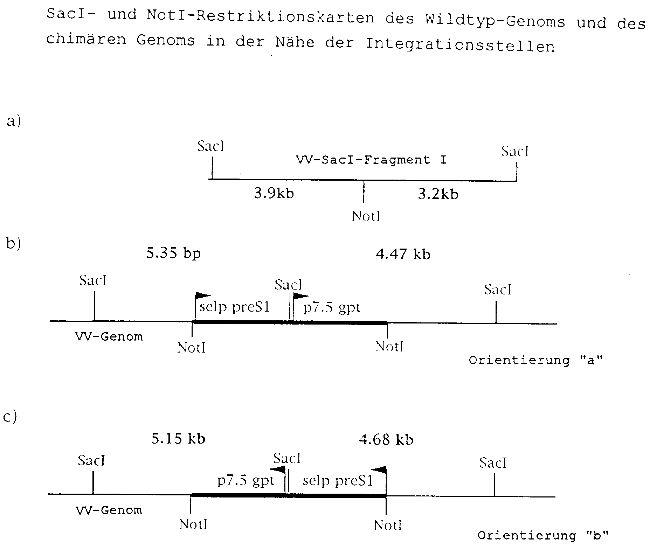

Fig. 3 - SacI-Restriktionsmuster von chimären preS1- oder preS1dMyr-Vaccinia-Viren. Die Figur zeigt die Größe der SacI-Fragmente, die mit dem ³²P-markierten, 7,1 kb großen Vaccinia-Virus-SacI-Fragment "I" von chimären preS1- und preS1dMyr-Vaccinia-Viren hybridisieren. Pfeile zeigen die Transkriptionsrichtung von den Promotoren selP und P7.5.

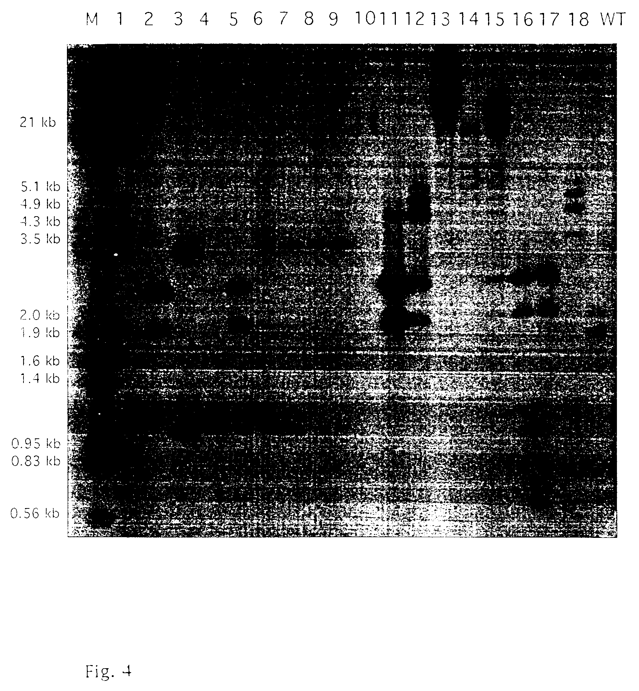

Fig. 4 - Muster der Hybridisierung mit einer ³²P-markierten, preS1-spezifischen Sonde von XbaI geschnittener genomischer DNA aus CV-1-Zellen, die mit chimären preS1- oder preS1dMyr-Vaccinia-Virus-Clonen infiziert wurden. CV-1-Zellen wurden mit chimären preS1- oder preS1dMyr-Vaccinia-Virus-Clonen oder Wildtyp-Vaccinia-Viren infiziert. Spuren 1 bis 10: mit XbaI geschnittene genomische DNA aus CV-1-Zellen, die mit vS1 #1-10 infiziert wurden. Spuren 11 bis 18: mit XbaI geschnittene genomische DNA aus CV-1-Zellen, die mit vS1dM #1-6, 9 und 10 infiziert wurden. WT = mit XbaI geschnittene genomische DNA aus CV-1-Zellen, die mit dem Wildtyp-Vaccinia-Virus infiziert wurden.

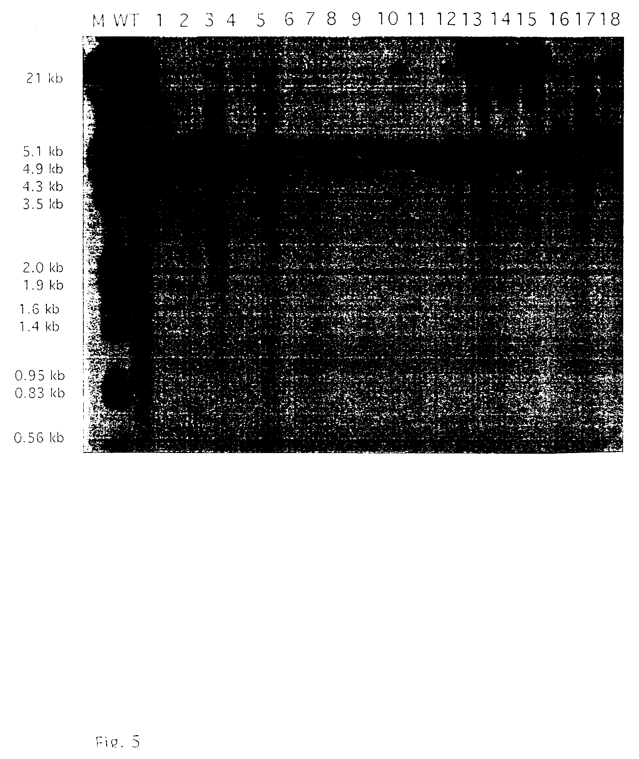

Fig. 5 - Muster der Hybridisierung mit einer ³²P-markierten, für das Vaccinia-Virus-SacI-Fragment 'I' spezifischen Sonde von SacI gescbnittener genomischer DNA aus CV-1-Zellen, die mit chimären vS1- oder vS1dM-Viren infiziert wurden. CV-1-Zellen wurden mit vS1, vS1dM oder Wildtyp-Vaccinia-Virus infiziert. Spuren 1 bis 10: mit SacI geschnittene genomische DNA aus CV-1-Zellen, die mit vS1 #1-10 infiziert wurden. Spuren 11 bis 18: mit SacI geschnittene genomische DNA aus CV-1-Zellen, die mit vS1dM #1-6, 9 und 10 infiziert wurden. WT = mit SacI geschnittene genomische DNA aus CV-1-Zellen, die mit Wildtyp-Vaccinia-Virus infiziert wurden.

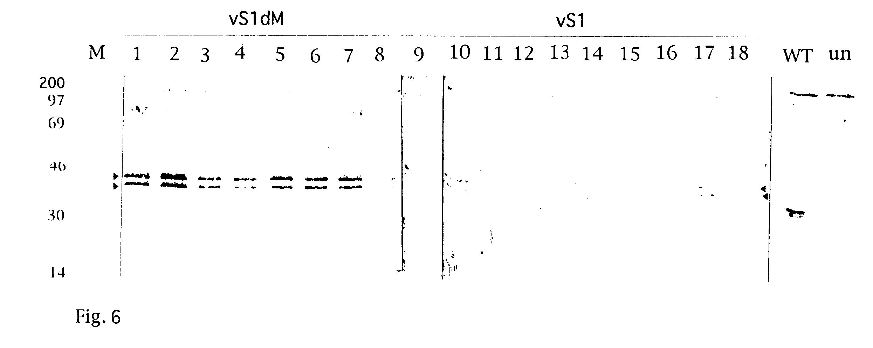

Fig. 6 - Western Blot-Analyse der Gesamtproteine von CV-1-Zellen, die mit chimären vS1- oder vS1dM-Vaccinia-Viren infiziert wurden. Pfeile bezeichnen die beiden Glycoformen des großen HBV-Proteins mit Molekulargewichten von 39 kDa bzw. 42 kDa. Spuren 1 bis 8: Lysate von CV-1-Zellen, die mit vS1dM #1-6, 9 und 10 infiziert wurden. Spuren 9 bis 18: Lysate von CV-1-Zellen, die mit vS1 #1-10 infiziert wurden. WT = Lysat aus CV-1-Zellen, die mit Wildtyp-Vaccinia-Virus infiziert wurden; un = Lysat aus uninfizierten CV-1-Zellen; M = Molekulargewichtsstandard in Kilodalton (kDa).

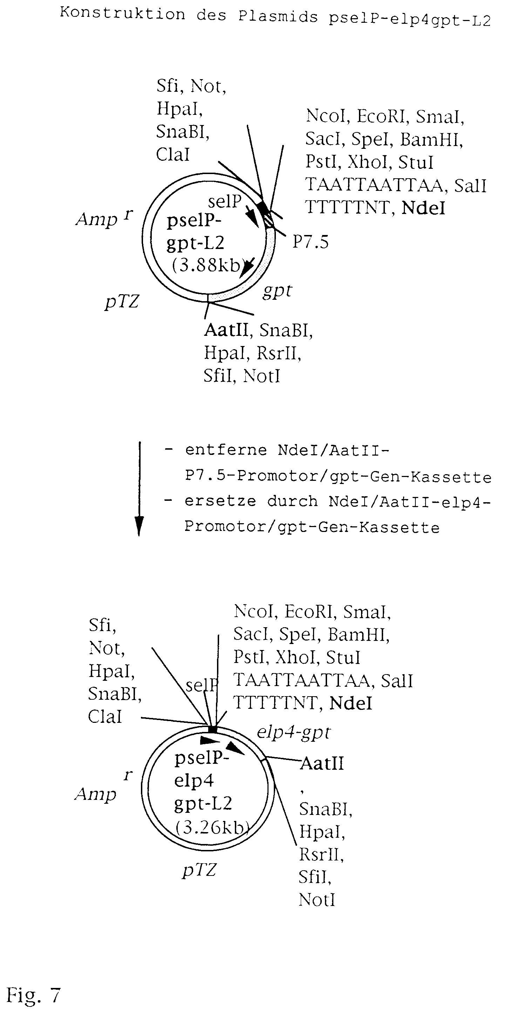

Fig. 7 - Konstruktion des Plasmids pselP-elp4gpt-L2. Der Vaccinia-Virus-Promotor P7.5 (P7.5) oder der synthetische frühe/späte Vaccinia-Promotor 4 (elp4) steuern das Xanthin-Guanin-Phosphoribosyltransferase-Gen (gpt-Gen) aus Escherichia coli. Pfeile bezeichnen die Richtung der Transkription.

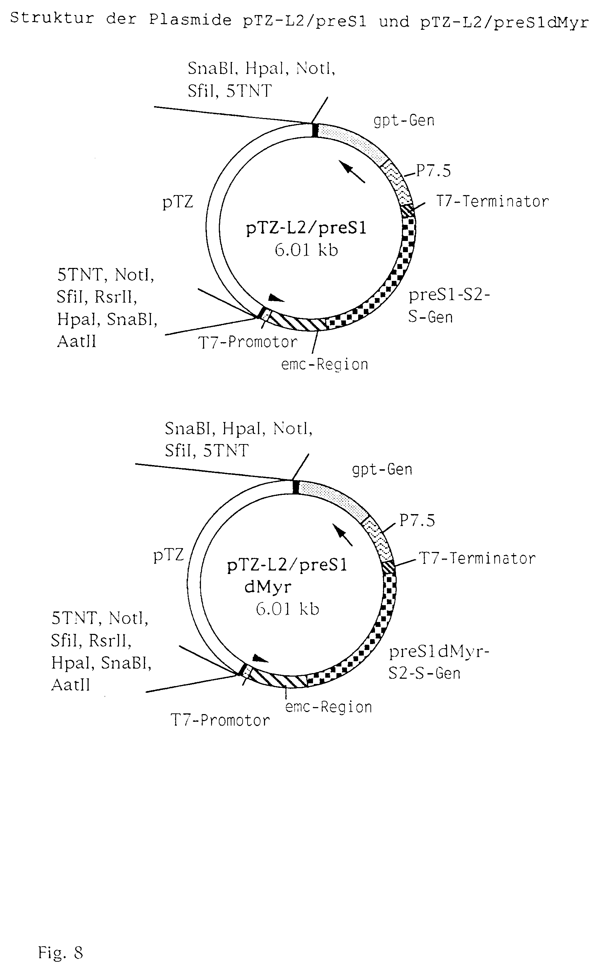

Fig. 8 - Struktur der Plasmide pTZ-L2/preS1 und pTZ-L2/preS1dMyr. Die Gene, die für das große HBV-Protein (preS1) oder seine demyristylierte Version (preS1dMyr) codieren, wurden unter die Kontrolle des Bakteriophage-T7-Promotors, der mit einer DNA-Kopie der 5'-nicht-codierenden Region des Encephalomyocarditis-Virus (emc) verknüpft war, gestellt. Für die weiteren Abkürzungen vergleiche die Legende von Fig. 7. Pfeile bezeichnen die Richtung der Transkription.

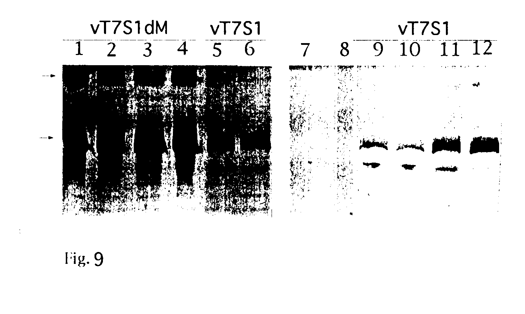

Fig. 9 - Western Blot der Gesamtproteine, die aus Zellen extrahiert wurden, die mit dem T7-Polymerase exprimierenden Virus vT7/selP und den Viren vT7S1 oder vT7S1dM coinfiziert wurden. Spuren 1 bis 4: Lysate aus CV-1-Zellen, die mit vT7S1dM #1-4 infiziert wurden. Spuren 5 und 6: Lysate aus CV-1-Zellen, die mit vT7S1 #1 und 2 infiziert wurden. Spur 7: Lysat aus CV-1-Zellen, die mit Wildtyp-Vaccinia-Viren infiziert wurden. Spur 8: Lysat aus uninfizierten CV-1-Zellen. Spuren 9 bis 12: Lysate aus CV-1-Zellen, die mit vT7S1 #3-6 infiziert wurden. Pfeile bezeichnen das monomere große Antigen (unterer Pfeil) und aggregierte Formen des Antigens (oberer Pfeil).

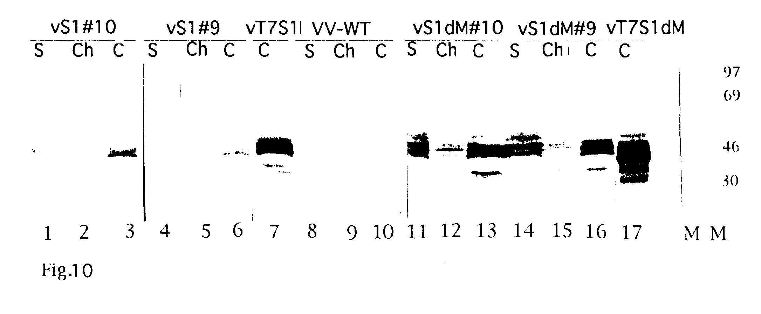

Fig. 10 - Western Blots der Gesamtproteine aus verschiedenen Zellinien, die mit chimären Viren infiziert wurden. Spuren 1 bis 3: Lysate aus SK-Hep1-Zellen (s), Chang-Leberzellen (Ch) oder CV-1-Zellen (C), die mit vS1 #10 infiziert wurden. Spuren 4 bis 6: Lysate aus SK-Hep1-Zellen, Chang-Leberzellen oder CV-1-Zellen die mit vS1 #9 infiziert wurden. Spur 7: Lysat aus CV-1-Zellen, die mit vT7S1 #1 infiziert wurden. Spuren 8 bis 10: Lysate aus Sk-Hep1-Zellen, Chang-Leberzellen oder CV-1-Zellen, die mit Wildtyp-Vaccinia-Viren infiziert wurden. Spuren 11 bis 13: Lysate aus SK-Hep1-Zellen, Chang-Leberzellen oder CV-1-Zellen, die mit vS1dM #10 infiziert wurden. Spuren 14 bis 16: Lysate aus SK-Hep1-Zellen, Chang-Leberzellen oder CV-1-Zellen, die mit vS1dM #9 infiziert wurden. Spur 17: Lysat aus CV-1-Zellen, die mit vT71dM #1 infiziert wurden.

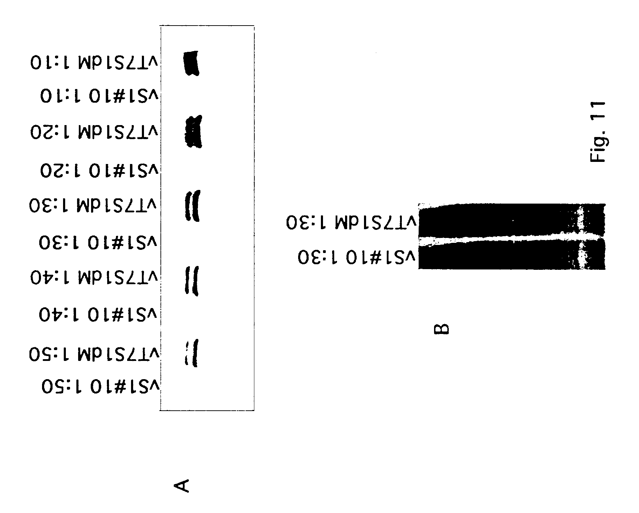

Fig. 11 - Western Blots der Gesamtproteine aus mit chimären Viren infizierten Zellen, die myristylierte und demyristylierte Antigene exprimieren. Fig. 11A zeigt die für preS1 spezifischen Signale der Immunanfärbung in einem Western Blot mit Lysat-Verdünnungen von 1:10, 1:20, 1:30, 1:40 und 1:50. Unterschiedliche Verdünnungen waren erforderlich, um das Signal der Immunanfärbung zu bestimmen, das für die Auswertung in einem densitometrischen Abtastsystem geeignet ist. Um gleiche Mengen an Gesamtprotein in den Lysaten zu zeigen, ist eine Anfärbung mit Coomassie-Blau der Verdünnung 1:30 der Lysate von CV-1-Zellen, die mit vS1#10 und vT7S1 dM infiziert waren, in Fig. 11B gezeigt.

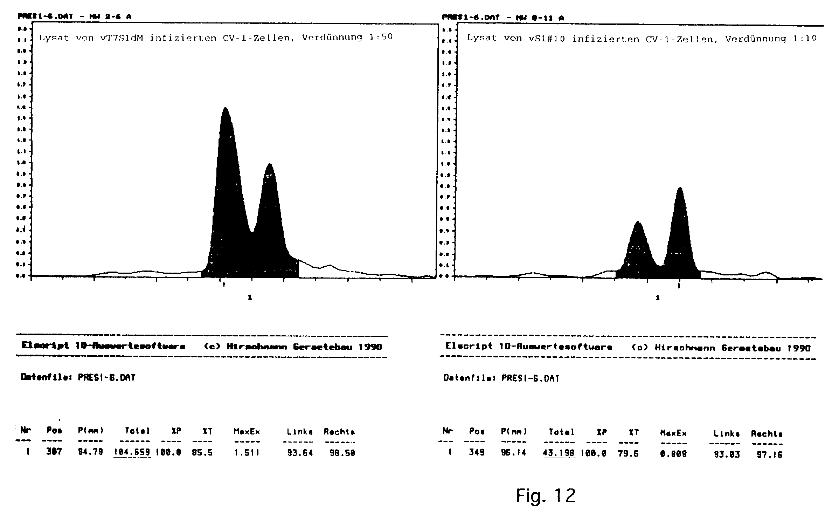

Fig. 12 - Ergebnisse der densitometrischen Abtastung von Western Blots der Gesamtproteine aus mit chimären Viren infizierten Zellen, die myristylierte und demyristylierte große Antigene exprimieren.Mit dem Computer erzeugte Grafiken der Ergebnisse der densitometrischen Abtastung von CV-1-Zellen, die mit vT7S1dM und vS1 #10 infiziert waren. Eine Verdünnung von 1:50 wurde für das T7/modifizierte Konstrukt eingesetzt, und eine Verdünnung von 1:10 wurde für das selP/Wildtyp-Konstrukt eingesetzt. Der schattierte Bereich unter der Kurve wurde berechnet und ist unter "gesamt" unten in jedem Diagramm angegeben.

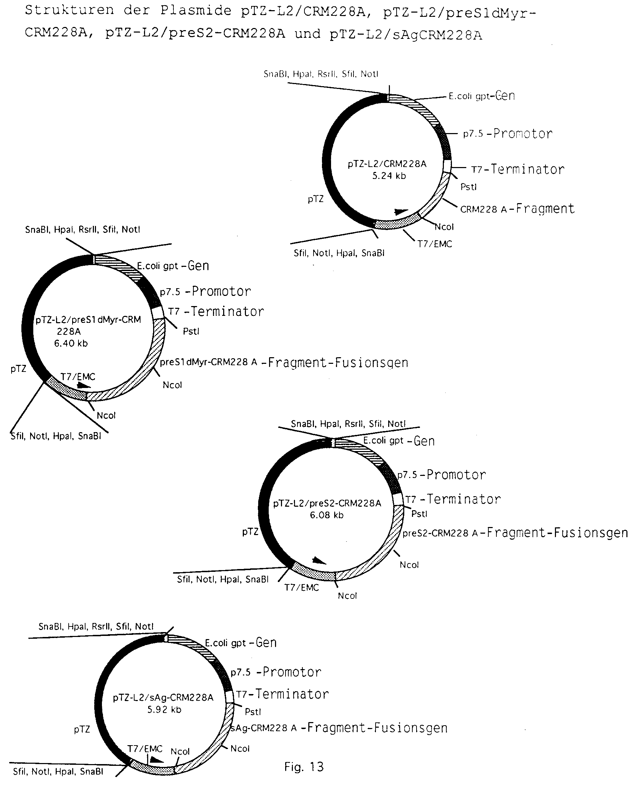

Fig. 13 - Struktur der Plasmide pTZ-L2/CRM228A, pTZ-L2/preS1dMyr-CRM228A, pTZ-L2/preS2-CRM228A und pTZ-L2/sAg-CRM228A. Die Nucleotidsequenz, die für das Fragment A des Corynebacteriophage beta tox 228-Gens codiert, wird im selben Leseraster als ein NcoI/PstI-Fragment mit den vollständigen offenen Leserahmen des HBV ayw-preS1-preS2-S-sAg-Gens, des preS2-S-sAg-Gens und des S-sAg-Gens fusioniert. Die Fusionsgene werden unter der transkriptionalen Steuerung eines modifizierten Bacteriophage T7-Promotors angeordnet. Die Richtung der Transkription ist durch Pfeile angegeben.

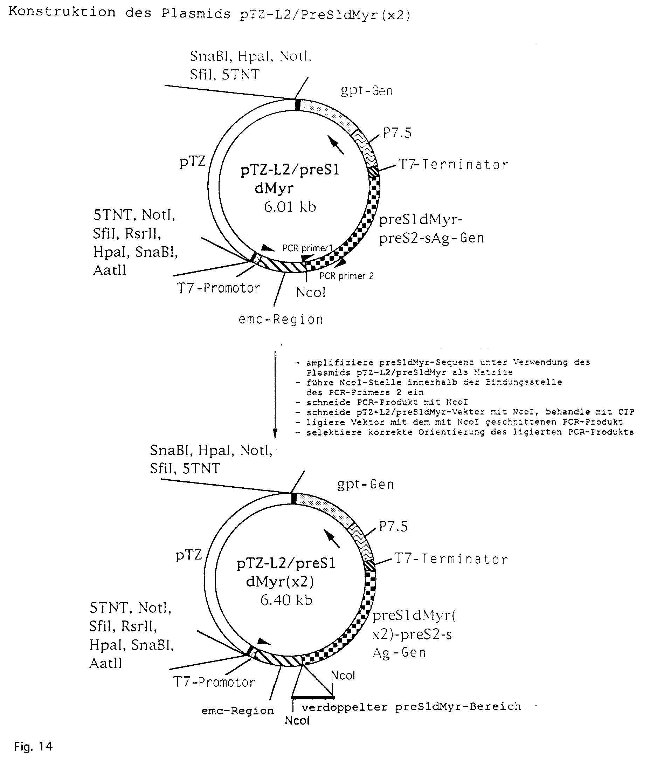

Fig. 14 - Konstruktion des Plasmids pTZ-L2/preS1dMyr(x2). Die Nucleotidsequenz des preS1-Teils (Nucleotide 1-324) des preS1dMyr-preS2-sAg-Gens wurde unter Verwendung des Plasmids pTZ-L2/preS1dMyr als Matrize und spezifischer PCR-Primer amplifiziert. Das PCR-Produkt wurde als ein NcoI-Fragment in einen mit NcoI geschnittenen und mit alkalischen Phosphatasen behandelten pTZ-L2/preS1dMyr-Vektor cloniert. Das preS1-preS1-preS2-sAg-Gen ist unter der transkriptionalen Steuerung eines modifizierten Bacteriophage T7-Promotors angeordnet. Die Position der PCR-Primer und die Richtung der Transkription sind durch Pfeile angegeben.

Ein rekombinantes Nucleotidmolekül wird konstruiert, das die Sequenz einschließt, die für die Struktur der preS1-Region des großen HBV-Antigens codiert. Zur Isolierung des Strukturgens für das große Antigen stellt Dane-Partikel-DNA eine leicht verfügbare Quelle dar, aus der Komponenten des HBV-Genoms unter Anwendung von Standardmethoden, wie sie dem Fachmann bekannt sind, isoliert werden können. Im allgemeinen umfaßt die Konstruktion die Isolierung der Dane-Partikel-DNA, das Schneiden der DNA mit Enzymen, die Identifizierung von DNA-Fragmenten, die das Strukturgen für das große Antigen enthalten, die Ligation des isolierten Gens mit einem Clonierungsvektor, die Rekombination eines Teils des Clonierungsvektors unter Einschluß der preS1-Region in einen Vaccinia-Virusvektor und die Infektion eines geeigneten Wirts mit dem chimären Vaccinia-Vektor. Der chimäre Vaccinia-Vektor ist zur Replikation in dem Wirt und zur Expression sowohl von Vaccinia-Genen als auch von Nicht-Vaccinia-Genen imstande. Infizierte Zellen exprimieren ein preS1 enthaltendes Protein.

Es kann auch eine chemische Synthese angewandt werden, um die erforderlichen DNA-Sequenzen zu erzeugen. Oligonucleotide können unter Verwendung einer Vorrichtung, wie der von Applied Biosystems, z.B. Modell 380A, oder durch chemische Techniken, wie der Methode von Matteucci et al., J. Am. Chem. Soc., Bd. 103 (1981), S. 3185-3191, synthetisiert werden. DNA-Sequenzen, die für die Proteine von HBV codieren, können aus Dane-Partikeln unter Anwendung von Methoden, wie denjenigen, die von Robinson, Am. J. Med. Sci., Bd. 270 (1975), S. 151-159, beschrieben wurden, isoliert werden.

Man nimmt an, daß die aminoterminale Myristylierungsstelle des großen HBV-Antigens zur Bindung des Proteins an Komponenten der Zelle, insbesondere der Zellmembran, führt. In unerwarteter Weise scheint es nun so, daß die Umwandlung von G in C am 5. Nucleotid der für preS1 codierenden Region, mit der die Zerstörung der Myristylierungsstelle beabsichtigt war, auch zu einem größeren translationalen Wirkungsgrad und damit zu erhöhten Ausbeuten am großen Antigen führt; vgl. Figuren 11 und 12 sowie Beispiel 4.

Vorzugsweise enthält das chimäre Virus auch ein Markergen, und Zellen, die mit einem derartigen Virus infiziert sind, exprimieren ein selektierbares Markerprotein. Durch Addition eines Markergens an das chimäre Vaccinia-Virus zusammen mit weiteren Sequenzen von Interesse können Vektoren oder Zellen, die die Sequenzen tragen, identifiziert und von denjenigen Zellen oder Vektoren ohne diese Sequenzen getrennt werden. Diese Selektion wird durch Kultivieren der Zellen in einer Umgebung, die das Wachstum von Viren, die das Markergen aufweisen, begünstigt, durchgeführt.

Gemäß der vorliegenden Erfindung wird das Strukturgen unter der Steuerung eines Promotors angeordnet. Das Vaccinia-Virus weist einzigartige transkriptionale regulatorische Signale auf, die von der viralen RNA-Polymerase erkannt werden. Daher werden definierte Vaccinia-Promotorsequenzen üblicherweise mit codierenden Sequenzen von Interesse ligiert. Ein starker Vaccinia-Promotor, wie selP, wird in diesem Zusammenhang bevorzugt.

Die vorliegende Erfindung faßt jedoch auch die Verwendung von Nicht-Vaccinia-Virus-Promotoren ins Auge. Der Promotor der Wahl stammt aus dem Bacteriophagen T7. Es ist gezeigt worden, daß das Vaccinia-Virus-Bacteriophage T7-Hybridexpressionssystem die Expression mehrerer fremder Gene wesentlich verstärkt; Elroy-Stein et al., 1989. Da die transkriptionale Maschinerie des Vaccinia-Virus auf diesen Promotor nicht wirkt, stellt die vorliegende Erfindung auch eine T7-RNA-Polymerase bereit. Das Strukturgen wird also funktionell mit einem regulatorischen Element aus dem Phagen verknüpft, wobei eine Phagen-Polymerase auf das regulatorische Element wirkt. Gemäß einer bevorzugten Ausführungsform sind das Promotor/großes HBV-Antigen-Gen und das T7-RNA-Polymerase-Gen in verschiedenen chimären Vaccinia-Viren enthalten und werden durch Coinfektion der gleichen Zelle in Kontakt gebracht.

Eine "Kassette" ist eine kovalent verknüpfte Reihe von DNA-Codons, die für mehrere nicht-überlappende Proteine codiert. In der vorliegenden Erfindung umfaßt eine Kassette ein preS1 enthaltendes Gen, und sie kann auch für ein Markergen codieren. Die Kassette enthält auch eine Promotor-Sequenz, die von dem T7-Phagen stammt. Normalerweise werden Kassetten von singulären Restriktionsstellen flankiert, die ein Ausschneiden aus einer Reihe von Clonierungsvektoren und eine Rekombination in eine Reihe von Clonierungsvektoren, in diesem Fall gentechnisch veränderte Vaccinia-Viren, erlauben.

Ein Clonierungsvektor wird als intermediärer Träger des isolierten Gens verwendet. Vorzugsweise enthält der Clonierungsvektor einen Promotor und ein Markergen, die verwendet werden können, um eine Kassette für die direkte Übertragung in einen Empfänger, wie das Vaccinia-Virusgenom, zu erzeugen. Die Einverleibung der Kassette in die Vaccinia-Sequenzen erfordert das Schneiden sowohl des preS1 enthaltenden Clonierungsvektors als auch des viralen Genoms, wobei letzteres im allgemeinen an einer bevorzugten Stelle relativ zu den Vaccinia-Virusgenen geschnitten wird. Die nächste Stufe ist die Ligation der ausgeschnittenen oder linearisierten Kassette mit dem Vaccinia-Genom. Dies führt zur Bildung eines linearen chimären viralen Genoms. Sodann wird die ligierte DNA in Zellen transfiziert, die mit einem zweiten Vaccinia-Virus infiziert sind, das das Packen der chimären DNA in ein infektiöses virales Partikel unterstützt.

Die Bildung eines preS1 enthaltenden Proteins gemäß der vorliegenden Erfindung umfaßt die Stufen des (i) Antigens einer Zellkultur und (ii) Infizierens der Kultur mit einem oder mehreren chimären Vaccinia-Viren. Die Zellen müssen permissiv für die Replikation von Vaccinia sein und können Säuger-Nierenzellinien, wie Vero (ATCC #CCl81) und CV-1 (ATCC #CCl70), sowie Leberzellinien, wie SK-HEP-1 (ATCC #HTB52) und Chang (ATCC #CCl13), umfassen.

Ein Vorteil des Vaccinia-Virussystems besteht in dem weiten Wirtszellbereich unter Einschluß von Leberzellen, Nierenzellen und vielen anderen Säugerzellinien. Diese Eigenschaft wird bis jetzt nicht für die Expression von rekombinanten HBV-Oberflächenglycoproteinen genutzt. Da die posttranslationale Prozessierung und Modifikation wichtige Aspekte der immunologischen Eigenschaften eines Antigens sind, beinhaltet das günstigste Expressionssystem für diese Art von Impfstoff eine Säugerzelle und insbesondere Leber- und Nierenzellen.

Bei einer Durchsicht des Standes der Technik muß die "Qualität" des Antigens, das aus bestimmten Wirtszellen erhalten wurde, im Hinblick auf mögliche Impfstoffe in Frage gestellt werden. Ohne eine geeignete Proteinprozessierung sind selbst große Mengen an Antigen von geringem Wert. Daher sind gemäß der vorliegenden Erfindung die Wirtszellen nicht nur permissiv, sondern repräsentieren auch die Arten von Zellen, in denen HBV normalerweise repliziert wird. Sie sind daher imstande, HBV-Antigene zu bilden, die stark denjenigen ähneln, die während einer natürlichen Infektion gebildet werden. Die kultivierten Zellen sollten imstande sein, für posttranslationale Modifikationen zu sorgen, die es rekombinanten Antigenen erlauben, natürliche, antigene und immunogene Epitope zu zeigen. Zellen, die ihren Ursprung in Leber und Niere haben, werden in dieser Hinsicht am stärksten bevorzugt.

Kulturen werden unter Bedingungen inkubiert, die die Expression der rekombinanten Proteine optimieren. Variablen, die optimiert werden können, umfassen, ohne Beschränkung hierauf, Temperatur, pH-Wert, CO₂-Konzentration sowie das Vorhandensein von Serumproteinen, Wachstumsfaktoren und Nährstoffen. Üblicherweise ist auch ein selektiver Faktor, der das Wachstum von Viren begünstigt, die die rekombinanten Gene tragen, eingeschlossen. Die Expression eines Proteins ist üblicherweise optimal, wenn eine maximale Menge an Protein gebildet worden ist. Infizierte Zellen können mechanisch, chemisch oder thermisch aufgebrochen werden, um die Ausbeute an Produkt zu erhöhen.

Wahlweise wird das rekombinante Produkt weiter gereinigt, wie es für eine gegebene Anwendung erforderlich ist. Verfahren zur Reinigung von Polypeptiden aus Zellkulturen sind dem Fachmann bekannt. Techniken, die angewandt werden können, umfassen Salzextraktion, Gradientenzentrifugation, Gelfiltration, Gelchromatographie, Ultrafiltration, Elektrophorese, Ionenaustausch und dergl.

Nicht alle Proteine als solche erweisen sich als wirksame Immunogene. Um die Immunogenizität zu erhöhen, welche inhärente Immunogenizität ein spezielles Protein auch immer haben mag, ist es oftmals ratsam, das Protein von Interesse an einen Immunopotentiator zu koppeln. Es gibt mehrere Klassen von Immunopotentiatoren unter Einschluß von auf Öl basierenden Trägern (Freund-Adjuvans), Aluminium-Trägersubstanzen (Alum), Toxinen (Lipid A) und Proteinen (Interleukine).

Gemäß einer speziellen Ausführungsform sind die Immunopotentiatoren Toxine, die chemisch oder, im Fall von Proteintoxinen, genetisch mit den Immunogenen verknüpft werden können. Mögliche Toxine für die Verwendung in derartigen Konstrukten umfassen, ohne Beschränkung hierauf, Ricin, Abrin, Pertussis-Toxin, Shigella-artiges Toxin, Cholera-Toxin, Staphylococcus-Exotoxin A, wärmestabiles und wärmelabiles Enterotoxin und Pseudomonas-Exotoxin A. In einigen Fällen ist es günstig, die Toxizität des Toxinteils durch chemische oder genetische Maßnahmen zu verringern; Revillard et al., Devel. Biol. Stand., Bd. 77 (1992), S. 31; Perentesis et al., Biofactors, Bd. 3 (1992), S. 173; Haslov et al., Immunobiology, Bd. 183 (1991), S. 40-54; Hussain et al., Nat. Immun. Cell Growth Reg., Bd. 8 (1989), S. 231-244.

Es wird hier zum ersten Mal gezeigt, daß Diphtherie-Toxin wirksam hinsichtlich einer Immunopotenzierung eines Immunogens ist, an das es gekoppelt ist. Bisher ist über die Verwendung von Diphtherie-Toxin in Fusionskonstrukten als ein Immunotoxin berichtet worden; Strom et al., Ann. N.Y. Acad. Sci., Bd. 636 (1981), S. 233-250; Shaw et al., J. Biol. Chem., Bd. 266 (1991), S. 21118-21124; Williams et al., J. Biol. Chem., Bd. 265 (1990), S. 11885-11889. Immunotoxine unterscheiden sich von Immunopotentiatoren jedoch dadurch, daß es sich bei ersteren um ein Toxin/Antikörper-Fusionsmolekül handelt, das selektiv Zielzellen zerstört, während letzteres ein Toxin oder ein Toxin/Protein-Fusionsmolekül ist, wobei der Toxinteil die Immunogenizität des Antigenteils verstärkt. Über die Verwendung von Toxinen, die von Diphtherie-Toxin verschieden sind, als Immunopotentiatoren, ist berichtet worden; Cholera-Toxin: Hussain et al. (1989); Pertussis-Toxin: Halsov et al., 1991.

Zwei Verfahren zur Kopplung sind für Proteintoxine möglich. Erstens können chemische Vernetzungsmittel verwendet werden, um das Proteinimmunogen physikalisch an einen Proteinimmunopotentiator anzuheften. Die Verfahren für das chemische Koppeln sind dem Fachmann bekannt; vgl. z.B. die Artikel von Feeney, Int. J. Peptide Protein Res., Bd. 29 (1987), S. 145-161; Peters und Richards, Ann. Rev. Biochem., Bd. 46 (1977), S. 523 ff; Erlanger, Meth. Enzymol., Bd. 70 (1980), S. 85ff; Han et al., Int. J. Biochem., Bd. 16 (1984), S. 129ff. Zweitens können Nucleinsäuresequenzen, die für ein Proteinimmunogen codieren, an Nucleinsäuresequenzen, die für den Immunopotentiator codieren, im gleichen Leseraster angehängt werden. Die Expression des erhaltenen "Hybrid-Gens" führt zu einem einzelnen Polypeptid, das sowohl Immunogen- als auch Immunopotentiator-Domänen enthält.

Ein weiteres Verfahren zur Erhöhung der Immunogenizität der preS1-Proteinsequenzen besteht darin, eine größere wirksame Dosierung der Epitope, die darin enthalten sind, bereitzustellen. Ein Weg, auf dem dies erzielt werden kann, ist die Verdopplung der preS1-Sequenzen im großen HBV-Antigen. Durch Insertion mindestens einer zweiten Kopie von preS1-codierenden Sequenzen in das preS1 enthaltende Konstrukt weist das exprimierte Protein davon mindestens zweimal so viele preS1-B- und T-Zell-Epitope auf. Diese höhere Epitopdichte kann dazu beitragen, die anti-preS1-Antwort zu stimulieren.

Das Gesamtergebnis der vorstehend genannten Verfahren zur Herstellung von HBV-preS1 enthaltenden Antigenen besteht in der Erzeugung eines geeigneten Materials für eine verbesserte HBV-Vaccine. Ein erheblicher Anteil (7 bis 15 %) der Population spricht nicht auf herkömmliche HBV-Impfstoffe an, die keine preS1-Regionen enthalten. Die Fähigkeit, Personen mit Antigenen zu immunisieren, die diese Region, die in einer Weise, die mit der natürlichen Infektion übereinstimmt, prozessiert wurde, enthält, erlaubt es, eine größere Anzahl an Personen vor HBV-Infektionen zu schützen.

Antikörper, die gegen das rekombinante, preS1 enthaltende Protein gerichtet sind, gehören ebenfalls zur vorliegenden Erfindung. Mindestens drei hauptsächliche Hindernisse bestehen, die die Bildung von HBV-preS1-spezifischen Antikörpern erschwert haben. Erstens waren große Mengen dieses Proteins nicht allgemein verfügbar. Zweitens verdünnte das Gemisch aus kleinen, mittleren und großen Antigenen, wenn große Mengen an rekombinant produzierten HBV-Antigenen verfügbar waren, den Anteil der preS1-Regionen. Und drittens wurden rekombinante preS1-Polypeptide bisher nicht hergestellt, die den posttranslationalen Modifikationen entsprachen, die bei natürlich infizierten Zellen festgestellt werden. Die vorliegende Erfindung stellt große Mengen an preS1 enthaltenden Antigenen bereit, die von Zelltypen prozessiert werden, die normalerweise mit HBV infiziert werden. Daher wird die Möglichkeit für die Herstellung sowohl polyklonaler als auch monoklonaler Antikörper, die sich für Nachweis, Therapie und Verhinderung von HBV-bezogenen Erkrankungen eignen, wesentlich verbessert.

Eine spezielle Verwendung von HBV-spezifischen monoklonalen Antikörpern ist die Verwendung als Immunotoxine. Immunotoxine sind Hybridproteinmoleküle, die durch die Fusion eines Antikörpers und eines Toxins erzeugt werden und verwendet werden können, um selektiv bestimmte Zellpopulationen zu zerstören. Die starke zellabtötende Wirkung eines Toxins wird mit der hervorragenden Spezifität von monoklonalen Antikörpern verbunden, wodurch eine selektive Zerstörung von Zielzellen bewirkt wird, während andere Zellen nicht betroffen sind. Beispiele für derartige Toxine umfassen, ohne Beschränkung hierauf, Ricin, Abrin, Pertussis-Toxin, Shigella-artiges Toxin, Cholera-Toxin, Staphylococcus-Exotoxin A, wärmestabiles und wärmelabiles Enterotoxin, Pseudomonas Exotoxin A und Diphtherie-Toxin. In einigen Fällen ist es vorteilhaft, die Toxizität des Toxinteils durch chemische oder genetische Maßnahmen zu verringern.

Die Fusion von Toxinen an Antikörper kann chemisch oder, im Fall von Proteintoxinen, genetisch erfolgen. Proteintoxingene können im Leseraster mit einem Einzelketten-Antikörper-Gen fusioniert werden (leichte Kette/schwere Kette-Hybride; vgl. Huston et al., Proc. Natl. Acad. Sci. USA, Bd. 85 (1988), S. 5879-5883, und Bird et al., Science, Bd. 242 (1988), S. 423-426). Alternativ kann der Einzelketten-Antikörper-Teil durch andere Bindungsproteine, wie Rezeptoren oder Rezeptorliganden, z.B. Il-2, ersetzt werden.

Um eine Prävention einer HBV-Erkrankung zu erreichen, wird ein Impfstoff in Betracht gezogen, der mindestens die preS1-Region von HBV, die nach der in der vorliegenden Anmeldung beschriebenen rekombinanten Technik hergestellt wurde, umfaßt. Der Impfstoff umfaßt in einem pharmazeutisch verträglichen Träger eine wirksame Menge der preS1 enthaltenden Proteine, die durch das rekombinante Expressionssystem in mit chimären Vaccinia-Viren infizierten Zellen hergestellt werden und eine geeignete Immunogenität aufweisen. Eine geeignete Immunogenität bedeutet, daß das Polypeptid imstande ist, eine Immunantwort hervorzurufen, die gegen eine natürliche HBV-Infektion schützt.

Zum Nachweis einer HBV-Erkrankung wird ein Reagenzsatz, der einen Behälter und (i) mindestens einen Antikörper, der gegen ein preS1 enthaltendes Molekül gerichtet ist, oder (ii) ein preS enthaltendes Molekül enthält, in Betracht gezogen. Am Nachweis eines preS1 enthaltenden Moleküls, das durch eine natürliche Infektion gebildet wurde, sind die erfindungsgemäßen Antikörper beteiligt. Am Nachweis von preS1-spezifischen Antikörpern, die von einem Wirt gegen eine natürliche Infektion oder eine Immunisierung gebildet wurden, sind die erfindungsgemäßen, preS1 enthaltenden Moleküle beteiligt. In beiden Fällen wird ein Antikörper, der mit einer chromatographischen, fluorometrischen oder sonstigen nachweisbaren Markierung versehen ist, verwendet, um entweder das preS1 enthaltende Protein oder einen preS1-spezifischen Antikörper nachzuweisen. Vorzugsweise umfaßt der Reagenzsatz auch eine positive Kontrolle für die Bindung eines preS1 enthaltenden Moleküls an einen preS1-spezifischen Antikörper oder umgekehrt sowie die Nachweisreaktion.

Die vorliegende Erfindung betrifft auch ein Verfahren zur Herstellung von proteinartigen Partikeln, die eine HBV-Antigenität und/oder HBV-Immunogenizität zeigen. Das Kultivieren von infizierten Zellen führt zur Bildung von proteinartigen Partikeln. Die Partikel enthaltenden Zellen können dann gesammelt werden. Gemäß bevorzugter Ausführungsformen werden die Partikel aus den Zellen unter Anwendung einer Reihe von Verfahren, die dem Fachmann bekannt sind, isoliert.

### Beispiel 1 - Konstruktion von Klonierungsvektoren

pselP-gpt-L2/preS1. Das 1447 Basenpaare umfassende Fragment NsiI (Position 1070 im HBV ayw-Genom)/BstEII (Position 2817 im HBV ayw-Genom) wurde aus dem Plasmid pTHBV isoliert; Christman et al., 1982. Überhänge, die aus dem Restriktionsendonucleaseverdau resultierten, wurden unter Verwendung von T4-DNA-Polymerase entfernt. Das Fragment mit stumpfen Enden wurde in den mit SmaI geschnittenen und dephosphorylierten Vektor pTM3 (Moss et al., 1990) kloniert, was zu dem intermediären Plasmid pTM3/preS1A führte, das ein korrekt orientiertes Insert trug. Der bakterielle Klon, der das Plasmid pTM3/preS1A trug, wurde mit einem M13-Helferphagen (Stratagene, Heidelberg, BRD) infiziert, um einzelsträngige (ss) Plasmid-DNA zu erhalten. Diese ss-DNA wurde verwendet, um eine NcoI-Schnittstelle am preS1-ATG-Startcodon mittels gezielter Mutagenese zu erzeugen (Mutagenese-Oligonucleotid: 5'-GGA AAG ATT CTG CCC CAT GGT GTA GAT CTT GTT-3'). Das intermediäre Plasmid wurde als pTM3/preS1B bezeichnet. Diese Manipulation erlaubte die Entfernung eines 40 bp umfassenden NcoI-Fragments, das 5'-nichtkodierende Sequenzen aus pTM3/preS1B enthielt. Der verbleibende Vektor wurde gereinigt und über die NcoI-Stellen erneut ligiert, was zu pTM3/preS1C führte. Das 1418 bp umfassende NcoI/PstI-Fragment, das das vollständige preS1-S2-S-sAg-Gen ohne 5'-nichtkodierende Sequenzen enthielt, wurde aus dem Plasmid pTM3/preS1C isoliert und mit dem mit NcoI/PstI geschnittenen pselP-gpt-L2-Vaccinia-Virus-Klonierungsvektor ligiert; vgl. US-Seriennr. 07/750 080. Das erhaltene Plasmid wurde als pselP-gpt-L2/preS1 bezeichnet.

pselP-gpt-L2/preS1dMyr. Einzelstrangige DNA des Plasmids pTM3/preS1A wurde verwendet, um eine NcoI-Schnittstelle am preS1-ATG-Startcodon durch gezielte Mutagenese unter Verwendung eines Mutagenese-Oligonucleotids 5'-GGA AAG ATT CTG CGC CAT GGT GTA GAT CTT GTT-3' zu erzeugen, das gleichzeitig ein Cytocin an Position 2 des zweiten Codons einführte, was ein Glycin am N-Terminus des offenen Leserahmens für preS1-S2-S in ein Alanin änderte (pTM3/preS1dMyrA). Das erhaltene 40 Basenpaare umfassende NcoI-Fragment, das 5'-nichtcodierende Sequenzen enthielt, wurde entfernt, und der verbleibende Vektor wurde gereinigt und über die NcoI-Stellen erneut ligiert, wobei man das Plasmid pTM3/preS1dMyrB erhielt. Das 1418 bp große NcoI/PstI-Fragment, das das vollständige modifizierte preS1-S2-S-Gen ohne 5'-nichtkodierende Sequenzen enthielt, wurde aus pTM3/preS1dMyrB isoliert und mit dem mit NcoI/PstI geschnittenen pselP-gpt-L2-Vaccinia-Virus-Klonierungsvektor ligiert; vgl. US-Seriennr. 07/750 080. Das erhaltene Plasmid wurde als pselP-gpt-L2/preS1dMyr bezeichnet.

pTZ-L2/preS1. Um das Plasmid pTZ-L2/preS1 zu erhalten, wurde die NcoI/PstI-Kassette aus pTM-3/preS1C mit dem mit NcoI/PstI geschnittenen pTZ-L2-Vektor ligiert; vgl. US-Seriennr. 07/750 080.

pTZ-L2/preS1dMyr. Um das Plasmid pTZ-L2/preS1dMyr zu erhalten, wurde die NcoI/PstI-Kassette aus pTM-3/preS1dMyrB mit dem mit NcoI/PstI geschnittenen pTZ-L2-Vektor ligiert; vgl. US-Seriennr. 07/750 080.

pselP-elp4gpt-L2. Um den Vaccinia-Virus-Promotor P7.5 aus pselP-gpt-L2 durch einen synthetischen schwachen frühen/späten Vaccinia-Virus-Promotor (elp4) zu ersetzen und um 3'-nichtcodierende Sequenzen aus der gpt-Gen-Kassette zu entfernen, wurde der offene Leserahmen des gpt-Gens in einer Polymerasekettenreaktion (PCR) aus pSV2gpt (ATCC #37145) unter Verwendung gpt-Gen spezifischen Oligonucleotiden (5'-ATC ATA TGA GGA TCC ATG AGC GAA AAA TAC ATC GTC ACC-3' und 5'-ATC CCG GGA CGT CAT AAA AAG ATT AGC GAC CGG AGA TTG GCG G-3') amplifiziert. Gleichzeitig wurden NdeI- und BamHI-Stellen am 5'-Ende und AatII- und SmaI-Stellen am 3'-Ende des gpt-Gens eingeführt. Die PCR-Produkte wurden mit NdeI/SmaI geschnitten und mit einem mit NdeI/SmaI geschnittenen pFS51-Vektor (EP-A-91 114 300.6) ligiert. Die elp4-Sequenz wurde als ein synthetischer NdeI/BamHI-Linker, der aus den hybridisierten Oligonukleotiden oelP4.1 (5'-TAT GTA AAA GTT GAA ATT ATT TTT TTA TGC TGT AAA TAA GTT AAC A-3') und oelP4.2 (5'-GAT CTG TTA ACT TAT TTA CTA GCA TAA AAA AAT AAT TTC AAC TTT TAC A-3'), bestand, stromaufwärts zum offenen Leserahmen des gpt-Gens eingeführt, wobei man das Plasmid pelP4-gpt erhielt. Die elp4-gpt-Gen-Kassette wurde aus dem Plasmid pelP4-gpt mit NdeI und AatII ausgeschnitten und mit NdeI/AatII geschnittener und gereinigter pselP-gpt-L2-Vektor-DNA ligiert, was zu dem Plasmid pselP-elp4gpt-L2 führte.

pselP-elp4gpt-L2/T7. Das 2,7 kb umfassende BamHI-Fragment mit dem Gen für die Bacteriophagen T7-RNA-Polymerase wurde aus dem Vaccinia-Virus vTF7/3 (Fuerst et al., 1986) isoliert und mit dem mit BamHI geschnittenen und dephosphorylierten pselP-elp4gpt-L2-Vektor ligiert. Ein Plasmidklon, der das T7-RNA-Polymerase-Gen in der korrekten Orientierung in Bezug auf den selP-Promotor trug, wurde isoliert. Dieses Plasmid wurde als pselP-elp4gpt-L2/T7 bezeichnet.

Um eine optimale Transkriptionseinheit zu konstruieren, wurden die 5'-nicht codierenden Sequenzen aus dem preS1-S2-S-Gen vollständig entfernt. Eine NcoI-Schnittstelle (5'-CCATGG-3') wurde um ATG-Startcodon durch gezielte Mutagenese eingeführt. Diese Manipulation erlaubte die direkte Fusion des ATG-Startcodons des entsprechenden Gens mit dem frühen/späten Vaccinia-Virus-Promotor (selP) oder dem Bacteriophage T7-Promotor. Die Plasmide pselP-gpt-L2/preS1 und pselP-gpt-L2/preS1dMyr (Fig. 1) wurden konstruiert, um das preS1-S2-S-sAg-Gen oder seine demyristylierte Version unter der Kontrolle des selP-Promotors zusammen mit dem E. coli gpt-Markergen unter der Kontrolle des schwachen Vaccinia-Promotors P7.5 in das Genom des Vaccinia-Virus einzuführen. Die doppelten Gen-Kassetten können aus den Plasmiden mit mehreren Restriktionsenzymen (z.B. NotI, SnaBI, SfiI) ausgeschnitten und mit den entsprechenden singulären Schnittstellen eines geeigneten Vaccinia-Virus-Vektors ligiert werden. Die Kassetten wurden mit NotI ausgeschnitten und mit Vaccinia-Virusarmen, die ebenfalls mit NotI geschnittenen worden waren, ligiert. Da die Not-Kassette, die aus pselP-gpt-L2/preS1 oder pselP-gpt-L2/preS1dMyr isoliert wurde, mit den mit NotI geschnittenen Vektorarmen in zwei verschiedenen Orientierungen ligiert werden kann, wurde ein diagnostischer Restriktionsendonucleaseverdau unter Verwendung der Enzyme XbaI und SacI durchgeführt. Das letztgenannte Enzym erlaubte parallel die Untersuchung auf das Vorhandensein von Vaccinia-Wildtyp-Virus unter Verwendung von SacI-geschnittener Wildtyp-DNA als Referenz. Der starke frühe/späte Promotor ist in US-Seriennr. 07/882 768 beschrieben.

In den Plasmiden pTZ-L2/preS1 und pTZ-L2/preS1dMyr (Fig. 8) sind das große HBV-Antigen-Gen oder seine demyristylierte Version unter der Kontrolle des Bakteriophagen T7-Promotors angeordnet. Die mit diesen Plasmiden konstruierten Viren wurden als vT7S1-Reihe bzw. vT7S1dM-Reihe bezeichnet.

pTZ-L2/CRM228A. Die Nucleotidsequenz, die für das Fragment A des Corynebacteriophage beta tox 228-Polypeptids (ohne Signalpeptidsequenz) codiert, wird in einer Polymerasekettenreaktion unter Verwendung der PCR-Primer 5' CCT TCA GCC CAT GCC ATG GCT GAT GAT GTT 3' und 5' A GCC ACC TAC TGC AGG CCT GAC ACG 3' amplifiziert. Der 5' Primer führt eine NcoI-Stelle ein, während der 3' Primer eine PstI-Stelle einführt. Das 606 bp umfassende PCR-Produkt wird mit NcoI und PstI geschnitten und in den mit NcoI/PstI geschnittenen pTZ-L2-Vektor (vgl. US-Seriennr. 07/914 738 cloniert, was zum Plasmid pTZ-L2/CRM228A führt.

pTZ-L2/preS1dMyr-CRM228A. Eine NcoI-Stelle wird am C-Terminus des offenen Leserahmens (ORF) von preS1dMyr unter Verwendung einzelsträngiger DNA aus pTZ-L2/preS1dMyr als Matrize und des mutagenen Oligonucleotids 5' TTT TGT TAG GGT TTC CAT GGA TAC CCA AAG ACA 3' eingeführt. Diese Manipulation ändert die beiden letzten Aminosäuren des offenen Leserahmens von preS1dMyr von Tyr-Ile in Ser-Met und führt einen zusätzlichen Glu-Rest ein. Das erhaltene Plasmid wird als pTZ-L2/preS1dMyrdTAA bezeichnet. Dieses Plasmid wird mit PstI linearisiert, partiell mit NcoI verdaut und mit dem NcoI/PstI-Insert des Plasmids pTZ-L2/CRM228A ligiert. Ein Plasmidklon, der das Gen für das CRM228 A-Fragment trägt, wird ausgewählt und als pTZ-L2-preS1-CRM 22817 bezeichnet.

pTZ-L2/preS2-CRM228A. Eine NcoI-Stelle wird am C-Terminus des offenen Leserahmens von preS2 unter Verwendung einzelsträngiger DNA aus dem Plasmid pTZ-L2/preS2 als Matrize und des mutagenen Oligonucleotids 5' TTT TGT TAG GGT TTC CAT GGA TAC CCA AAG ACA 3' eingeführt. Diese Manipulation ändert die beiden letzten Aminosäuren des ORF von preS2 von Tyr-Ile zu Ser-Met und führt einen zusätzlichen Glu-Rest ein. Das erhaltene Plasmid wird als pTZ-L2/preS2dTAA bezeichnet. Dieses Plasmid wird mit PstI linearisiert, partiell mit NcoI verdaut und mit dem NcoI/PstI-Insert des Plasmids pTZ-L2/CRM228A ligiert. Ein Plasmidklon, der das Gen für das CRM228 A-Fragment trägt, wird ausgewählt und als pTZ-L2/preS2-CRM228 A bezeichnet.

pTZ-L2/sAg-CRM228A. Eine NcoI-Stelle wird am C-Terminus des offenen Leserahmens von HBV-sAg unter Verwendung einzelsträngiger DNA aus dem Plasmid pTZ-L2/sAg als Matrize und des mutagenen Oligonucleotids 5' TTT TGT TAG GGT TTC CAT GGA TAC CCA AAG ACA 3' eingeführt. Diese Manipulation ändert die Aminosäuresequenz des ORF von HBV sAg von Tyr-Ile in Ser-Met und führt einen zusätzlichen Glu-Rest ein. Das erhaltene Plasmid wird als pTZ-L2/sAgdTAA bezeichnet. Dieses Plasmid wird mit PstI linearisiert, partiell mit NcoI verdaut und mit dem NcoI/PstI-Insert des Plasmids pTZ-L2CRM228A ligiert. Ein Plasmidklon, der das Gen für das CRM228 A-Fragment trägt, wird ausgewählt und als pTZ-L2/sAg-CRM228A bezeichnet.

pTZ-L2/preS1dMyr(x2). Die Nucleotidsequenz, die die Aminosäuren 1-108 des ORF von preS1dMyr umfaßt, wird in einer PCR-Reaktion unter Verwendung von pTZ-L2/preS1dMyr als Matrize und der Oligonucleotide 5' TTT TGG AAT ATA AAT AAG GC 3' und 5' ATT CCA CTC CAT GGC CTG AG 3' als PCR-Primer amplifiziert, wobei der letztgenannte Primer eine zweite NcoI-Schnittstelle in das PCR-Produkt einführt. Das 360 bp umfassende PCR-Produkt wird mit NcoI geschnitten und mit dem mit NcoI geschnittenen und mit alkalischer Phosphatase behandelten pTZ-L2/preS1dMyr-Vektor ligiert. Ein Plasmid, das das PCR-Produkt in der korrekten Orientierung relativ zum alten Leseraster des preS1-Gens trägt, wird ausgewählt und als pTZ-L2/preS1dMyr(x2) bezeichnet.

### Beispiel 2 - In vitro-Clonierung von chimären Vaccinia-Viren

Die NotI-Gen-Kassetten aus den preS1, preS1dMyr oder T7-Polymerase enthaltenden Plasmiden (pTZ-L2/preS1, pTZ-L2/preS1dMyr, pselP-gpt-L2/preS1, pselP-gpt-L2/preS1dMyr, pselP-elp4gpt-L2/T7) wurden isoliert und mit der mit NotI geschnittenen genomischen DNA aus dem Vaccinia-Virus-Wildtypstamm WR ligiert, wie es im wesentlichen in US-Seriennr. 07/750 080 beschrieben ist. Die Ligationskomponenten bestanden aus 1 µg mit NotI geschnittener genomischer Vaccinia-Virus-DNA, 500 ng NotI-Kassette, die aus den entsprechenden Plasmiden isoliert worden war, 5 Units T4-DNA-Ligase und Reaktionspuffer in einem Gesamtvolumen von 50 µl. Nach Inkubation für 16 Stunden bei 16°C wurde das Ligationsgemisch zusammen mit Calciumphosphat kopräzipitiert und zu subkonfluent gewachsenen CV-1-Zellen gegeben. Diese Zellen waren mit dem Geflügelpocken-Virusstamm HP1-441 (Mayr & Malicki, 1996) 2 Stunden zuvor mit einer multiplicity of infection, (moi; Viren pro Zelle) von 0,1 Plaque-bildenden Einheiten (pfu) pro Zelle infiziert worden. 5 bis 7 Tage nach der Infektion war die Zellmonolayer vollständig mit Vaccinia-Viren infiziert. Die Zellen wurden geerntet, und das Virus wurde aus den Zellfragmenten mittels Beschallung freigesetzt.

Die Selektion auf chimäre Vaccinia-Viren wurde durchgeführt, indem die Virusnachkommen in Gegenwart von 25 µg/ml Mycophenolsäure, 15 µg/ml Hypoxanthin und 125 µg/ml Xanthin titriert wurden. Unter diesen Bedingungen gewachsene Viren wurden weiter Plaque-gereinigt und für die Herstellung von "kleinen" und "großen" Virus-Stammlösungen verwendet. Kleine Virus-Stammlösungen, die aus einzelnen Virusplaques isoliert wurden, wurden auch unter selektiven Bedingungen gezüchtet. Infizierte Zellen aus kleinen Stammlösungen wurden in zwei Kulturen geteilt. Eine Kultur wurde zur Herstellung von großen Virus-Stammlösungen verwendet. Aus der anderen wurde DNA isoliert, mit verschiedenen Restriktionsendonucleasen verdaut und auf Nitrocellulosefilter übertragen. Diese Filter wurden mit spezifischen Sonden hybridisiert, um die Orientierung der NotI-Kassette im Genom des chimären Vaccinia-Virus und das Fehlen von Wildtyp-Vaccinia-Virus zu bestätigen.

Die XbaI-Fragmente aus genomischer DNA eines chimären preS1-Vaccinia-Virus, die mit einer ³²P-dCTP-markierte preS1-spezifischen Sonde hybridisieren, sollten eine Größe von 1,01 kb und 3,3 kb oder 1,9 kb und 2,41 kb, abhängig von der Orientierung der NotI-Kassette (Fig. 2) aufweisen, während die SacI-Fragmente aus dem gleichen chimären Virus, die mit den ³²P-dCTP-markierten, 7,1 kb großen Vaccinia-Virus-SacI-Fragment "I" hybridisieren, eine Größe von 5,35 kb und 4,47 kb oder 5,15 kb und 4,68 kb, abhängig von der Orientierung (Fig. 3), aufweisen sollten. In Fig. 2a sind die singuläre NotI-Schnittstelle und die flankierenden XbaI-Schnittstellen des Wildtyp-Vaccinia-Virusgenoms gezeigt. Die Integration der NotI-Kassette in die singuläre NotI-Schnittstelle in der Orientierung "a" führt zur Bildung von zwei neuen XbaI-Fragmenten mit einer Länge von 1,01 kb und 3,3 kb (vgl. Fig. 2b). Die Integration in der Orientierung "b" führt zu zwei neuen XbaI-Fragmenten mit einer Länge von 2,41 kb und 1,9 kb (vgl. Fig. 2c). In Fig. 3a sind die singuläre NotI-Schnittstelle und die flankierenden SacI-Schnittstellen im Wildtyp-Vaccinia-Virusgenom gezeigt. Die Integration der NotI-Kassette in die singuläre NotI-Schnittstelle in der Orientierung "a" führt zur Bildung von zwei neuen SacI-Fragmenten mit einer Länge von 5,35 kb und 4,47 kb (vgl. Fig. 3b). Die Integration in der Orientierung "b" erzeugt zwei neue XbaI-Fragmente mit einer Länge von 5,15 kb und 4,68 kb (vgl. Fig. 3c). Im Fall einer Kontamination von chimären Virus-Stammlösungen mit Wildtyp-Vaccinia-Virus-DNA sollte ein 7,1 kb großes SacI-Fragment ebenfalls mit der SacI-Sonde hybridisieren (Fig. 3a).

Die Hybridisierungsmuster der mit XbaI und SacI geschnittenen genomischen DNA aus 10 chimären preS1-Viren, nämlich vS1 #1-10, und 8 chimären preS1dMyr-Viren, nämlich vS1dM #1-6, 9 und 10, entweder mit der preS1-Sonde oder der Vaccinia-Virus-SacI Sonde-I sind in den Figuren 4 bzw. 5 gezeigt. In Fig. 4 wurde genomische DNA aus infizierten Zellpellets isoliert und mit XbaI geschnitten. DNA-Fragmente wurden auf einem 1 % DNA-Agarosegel aufgetrennt und vor der Hybridisierung mit Sonde auf Nitrocellulosefilter übertragen. Die Hybridisierung der XbaI-Fragmente zeigte, daß vS1 #1, 2, 4 und 5 (Fig. 4, Spuren 1, 2, 4 und 5) die NotI-Kassette in der Orientierung "b" aufwiesen und die Viren vS1 #3, 6-9, (Fig. 4, Spuren 3 und 6-9) in der Orientierung "a". Das Virusisolat vS1 #10 (Spur 10) zeigte ein schwaches Hybridisierungssignal im hochmolekularen Bereich.

Die Viren vS1dM #1, 2, 5, 6 und 9 (Fig. 4, Spuren 11, 12, 15, 16 und 17) hatten die NotI-Kassette in der Orientierung "b" inseriert, das Virusisolat vS1dM #10, Fig. 4, Spur 18) repräsentiert die Orientierung "a". Die chimären Viren vS1dM #3 und 4 (Fig. 4, Spuren 13 und 14) zeigten Hybridisierungssignale im hochmolekularen Bereich. Diese Art von Signal trat auch bei vS1dM #2 und 5 (Fig. 4, Spuren 12 und 15) und bei vS1dM #10 (Fig. 4, Spur 18) auf und deutet möglicherweise darauf hin, daß mehrfache Kopien der NotI-Kassette in das virale Genom integriert worden sind. Die genaue Struktur dieser Viren ist jedoch noch zu bestimmen.

In Fig. 5 wurde genomische DNA aus infizierten Zellen isoliert und mit SacI geschnitten. Die DNA-Fragmente wurden auf einem 1 % DNA-Agarosegel getrennt und vor der Hybridisierung mit der Sonde auf Nitrocellulose übertragen. Die Analyse der SacI-Fragmente, die mit der SacI Sonde-I hybridisierten, zeigte die erwarteten Muster der Orientierung "a" (4,47 und 5,35 kb) und der Orientierung "b" (4,68 kb und 5,15 kb). Außerdem ist das 7,1 kb umfassende Wildtyp-Vaccinia-Virus-SacI Fragment "I" (Fig. 5, Spur WT) in keinem der chimären Viren vorhanden. Die Möglichkeit einer Kontamination der chimären Viren mit Wildtyp-Vaccinia-Virus konnte daher ausgeschlossen werden.

Ein chimäres Vaccinia-Virus, das das T7-RNA-Polymerase-Gen trägt, nämlich vT7/selP, wurde durch Integration der NotI-Kassette aus dem Plasmid pselP-elp4gpt-L2/T7 in die NotI-Schnittstelle der Vacciniavirus genomischen DNA konstruiert. Dieses Plasmid wurde aus dem Ausgangsplasmid pselP-elp4gpt-L2 konstruiert, das eine verbesserte gpt-Gen-Kassette enthält. Ausgehend von dem Vaccinia-Virus in vitro-Klonierungsplasmid pselP-gpt-L2 (US-Seriennr. 07/750 080) wurde das Plasmid pselP-elp4gpt-L2 konstruiert, wie es in Fig. 7 gezeigt ist. In den bisherigen Vaccinia-Klonierungsplasmiden, die das gpt-Gen tragen, wird das Gen das für den dominanten Selektionsmarker kodiert durch den Vaccinia-Promotor P7.5 gesteuert, der auf einem 500 Basenpaare großen Vaccinia-DNA-Fragment lokalisiert ist. Dieses Fragment kann zu unerwünschten Rekombinationsereignissen mit zwei endogenen Vaccinia-Virus p7.5 Promotorsequenzen führen; Venkatesan et al., 1981. Um diese mögliche Art von Instabilität zu vermeiden, wurde die P7.5-gpt-Gen-Kassette (mit einer Größe von 1,1 kb) durch die elp4-gpt-Gen-Kassette (mit einer Größe von 0,5 kb) ersetzt. Die neue Gen-Kassette wies zusätzlich zu einem schwachen, nichthomologen synthetischen frühen/späten Promotor eine erheblich verringerte Größe auf. Das resultierende Plasmid, das den optimierten dominanten Marker und den starken synthetischen frühen/späten Promotor, gefolgt von einer multiple cloning site, aufwies, wurde als pselP-elp4gpt-L2 bezeichnet.

### Beispiel 3 - Expression des großen HBV-Antigens

Die Versuche wurden mit titrierten Stammlösungen des chimären Vaccinia-Viren durchgeführt. Zu diesem Zweck wurden CV-1-Zellen (ATCC CCL 70), SK-Hep1-Zellen (ATCC HTB 52) oder Chang-Leberzellen (ATCC CCL3), die bis zur Konfluenz in 10 cm Petrischalen gezüchtet worden waren (5x10⁶ Zellen), mit einer moi von 1 mit chimären preS1-Vaccinia-Viren, chimären preS1dMyr-Vaccinia-Viren oder chimären Vaccinia-Viren mit den preS1- oder preS1dMyr-Genen unter der Kontrolle des Bakteriophagen T7-Promotors infiziert. Mit den T7-preS1-Chimären infizierte Zellen wurden mit einer moi von 1 mit einer Vaccinia-Helfervirus-Chimäre koinfiziert, die das Gen für die Bakteriophagen T7-RNA-Polymerase unter der Kontrolle des selP-Promotors (vT7/selP) trug. Zur Kontrolle wurden Zellen mit vT7/selP allein infiziert, oder nach infiziert. Die Zellen wurden 3 Tage nach der Infektion geerntet. Infizierte Zellen wurden pelletiert und in 300 µl Natriumdodecylsulfat-Probenpuffer (SDS-Probenpuffer) resuspendiert. 10 bis 20 µl des Lysats wurden beschallt, gekocht und einer Elektrophorese auf einem 12 % SDS-Polyacrylamidgel unterworfen.

Um die Expressionsausbeute des großen Antigens bei allen ausgewählten Chimären zu bewerten, wurden Lysate von infizierten CV-1-Zellen in einem Western Blot analysiert. Fig. 6 zeigt die Expressionsausbeute in CV-1-Zellen, die mit acht vS1dM-Isolierungen (Fig. 6, Spuren 1 bis 8) oder 10 vS1-Isolierungen (Fig. 6, Spuren 9 bis 18) infiziert waren. Weder die Orientierung der NotI-Gen-Kassetten noch die unerwarteten Integrationsereignisse, die in Figuren 4 und 5 sichtbar sind, beeinflussen den Expressionswirkungsgrad der vS1dM-Viren (Fig. 6, Spuren 1 bis 8). Der direkte Vergleich der Expressionsausbeute der Zellen, die mit vS1dM-Viren (Fig. 6, Spuren 1 bis 8) infiziert wurden, mit vS1-Viren (Fig. 6, Spuren 9 bis 18) zeigt, daß die Modifikation der preS1-Genregion, d.h. die Deletion der Myristylierungsstelle, zu einem signifikanten Anstieg der Expression des großen Antigens führte. Da die gesamten zellulären Proteine in diesem Versuch analysiert wurden, hat diese Wirkung ihre Ursache nicht in einer besseren Löslichkeit des demyristylierten großen Antigens, sondern mit größerer Wahrscheinlichkeit in einer verbesserten Translation der preS1dMyr-Messenger-RNA (mRNA) oder alternativ in verbesserten posttranslationalen Prozessierungs- und Transport-Ereignissen. Das Molekulargewicht des rekombinanten großen HBV-Antigens entsprach dem bei der natürlichen HBV-Infektion synthetisierten. Die beiden Glycoformen mit Molekulargewichten von 39 kDa bzw. 42 kDa konnten klar unterschieden werden.

Für die Expressionsstudien mit dem Vaccinia-T7-System wurden CV-1-Zellen mit vT7S1- oder vT7S1dM-Viren und mit dem zweiten Virus, nämlich vT7/selP, das das Gen für die Bakteriophagen T7-RNA-Polymerase trug, koinfiziert. Lysate aus CV-1-Zellen, die mit vier verschiedenen Isolaten von vT7S1dM (Fig. 9, Spuren 1 bis 4) und 6 verschiedenen vT7S1-Viren (Fig. 9, Spuren 5 bis 6 und 9 bis 12) infiziert waren, wurden analysiert. Zur Kontrolle wurden auch Lysate von nachinfizierten CV-1-Zellen oder von CV-1-Zellen, die mit vT7/selP alleine infiziert wurden, analysiert (Fig. 9, Spuren 7 bzw. 8). Der direkte Vergleich der Expressionsausbeuten des großen Antigens und seiner demyristylierten Version, die mit diesem System erzielt wurden, bestätigte die mit den herkömmlichen Promotorkonstrukten erhaltenen Ergebnisse. CV-1-Zellen, die mit den vT7S1dM-Viren infiziert worden waren, bildeten signifikant mehr großes Protein als Zellen, die mit den vT7S1-Chimären infiziert wurden. Eine vorsichtige quantitative Bestimmung zeigte eine mindestens 5-fach höhere Produktionsrate des modifizierten großen Antigens gegenüber dem großen Wildtyp-Antigen.

### Beispiel 4 - Quantitative Bestimmung des großen Antigens mittels Western Blot-Analyse

Die Expressionsausbeute wurden in einem Western Blot verglichen, der im wesentlichen gemäß Towbin et al. (1979) mit den folgenden Modifikationen durchgeführt wurde. Bei dem ersten Antikörper handelte es sich entweder um ein Ziegenserum spezifisch für Hepatitis assoziierte Antigene (HAA), (Axell, Katalognr. AXL 683), oder um für die preS1-Region spezifische monoclonale Mäuse-Antikörper (Immunotech S.A., Katalognr. 0866), die in einer Verdünnung von 1:200 in einem Western Blot-Puffer (phosphatgepufferte Kochsalzlösung mit einem Gehalt an 0,1 % Tween 20) verwendet wurden. Bei dem zweiten Antikörper handelte es sich um ein mit alkalischer Phosphatase gekoppeltes Ziegen anti-Maus-Serum (Sigma Chemicals, Inc., Katalognr. A-4656) in einer Verdünnung von 1:1000. Das Nachweissystem (NBT und alkalische Phosphatase) stammte von Promega, Inc.

Die quantitative Bestimmung des großen HBV-Antigens wurde durch Vergleich der Western Blot-Banden durchgeführt. Als ein interner Standard wurde gereinigtes mittleres HBV ayw-Antigen, das im Vero-Zellen/Vaccinia-Virus-System exprimiert wurde, verwendet. Eine Verdünnungsreihe wurde mit dem gereinigten mittleren Antigen und mit Zubereitungen des großen Antigens durchgeführt. Die Verdünnungen wurden einer Elektrophorese auf einem 12 % SDS-Polyacrylamidgel unterworfen, auf Nitrocellulose übertragen und in einem Western Blot unter Verwendung von anti-HAA-Serum als einem ersten Antikörper und einem mit alkalischer Phosphatase konjugierten anti-Ziegen-IgG-Serum als einem zweiten Antikörper durchgeführt. Abhängig vom Zelltyp, den Zellkulturbedingungen und den verwendeten viralen Vektoren wurden 1 bis 10 µg großes Antigen/10⁶-Zellen nachgewiesen.

Um die Expressionsgrade des großen HBV-Antigens zu vergleichen, wurden CV-1-Zellen (eine Affen-Nierenzellinie), SK-Hep1-Zellen und Chang-Leberzellen (beides menschliche Leberzellinien) mit vS1 #9 (Orientierung: "a") und vS1 #10 (Orientierung: "b") oder mit vS1dM #9 (Orientierung: "b") und vS1dM #10 (Orientierung: "a", wahrscheinlich mehrere Inserts) infiziert. Parallel dazu wurden alle Zellinien mit Wildtyp-Vaccinia-Virus als Kontrolle infiziert. Fig. 10 zeigt die Ergebnisse der Expressionsstudie. Lysate aus SK-Hep1-Zellen (S), Chang-Leberzellen (Ch) und CV-1-Zellen (C), die mit vS1 #9 und vS1 #10, Wildtyp-Vaccinia-Virus und vS1dM #9 und vS1dM #10 infiziert waren, wurden verglichen. Die erhöhten Expressionsgrade der Chimären mit dem demyristylierten preS1-Gen (Fig. 10, Spuren 11 bis 16) waren erneut im Vergleich mit denjenigen des Wildtyp-preS1-Gens (Spuren 1 bis 6) klar sichtbar. Dieses Ergebnis zeigt, daß diese Wirkung nicht auf eine einzelne Zellinie beschränkt ist, sondern wiederholt in verschiedenen Zellinien beobachtet werden konnte.

Darüber hinaus exprimieren alle Zellinien die beiden authentischen molekularen Formen des großen Antigens, die in Fig. 6 beobachtet werden. Die Expressionsausbeute unterscheiden sich jedoch signifikant. Chang-Leberzellen (Fig. 10, Spuren 2, 5, 12 und 15) synthetisieren das große Antigen wesentlich schlechter als SK-Hep1-Zellen (Spuren 1, 4, 11 und 14) oder CV-1-Zellen (Spuren 3, 6, 13 und 16). Dieser Befund zeigt die Bedeutung der Auswahl der optimalen Zellinie.

Der direkte Vergleich der Expressionsausbeute ausgehend vom Bakteriophagen T7-Promotor gegenüber dem Vaccinia-Virus-selP-Promotor wurde parallel dazu durchgeführt. Zu diesem Zweck wurden Lysate von CV-1-Zellen, die mit vT7S1 bzw. vT7S1dM infiziert waren, ebenfalls in dem Western Blot, der in Fig. 10 gezeigt ist, analysiert. Der Unterschied der Expressionsausbeute des großen Antigens und seiner modifizierten Version wurde in einem Western Blot zu Vergleichszwecken analysiert. Die Expressionsausbeute des großen Antigens oder seiner modifizierten Version im T7-System waren mindestens 5-fach höher im Vergleich zu den Expressionsausbeuten, die im selP-Promotor-System gefunden wurden (Fig. 10, Spur 6, verglichen mit 7, oder Spur 16, verglichen mit 17).

Um die preS1-Expressionsausbeute des chimären Virus, das das preS1-Wildtyp-Gen unter der Steuerung des Vaccinia selP-Promotors trägt (vS1 #10, schwächste preS1-Expression; vgl. Fig. 10, Spur 3) mit dem chimären Virus, das das modifizierte preS1-Gen unter der Steuerung des Bakteriophagen T7-Promotors trägt (vT7S1dM, stärkste preS1-Expression; vgl. Fig. 10, Spur 17), genau zu vergleichen, wurden Verdünnungen von Zellysaten aus beiden Infektionen in einem Western Blot analysiert. Um die offensichtlichen Unterschiede, die in Fig. 11A ersichtlich sind, quantitativ zu bestimmen, wurden die Blots einer densitometrischen Analyse unterzogen.

Kulturen mit 1 x 10⁷ CV-1-Zellen wurden mit vLS1 #10 mit einer moi von 2 pfu/Zelle oder mit vT7S1dM und dem Helfervirus vT7/selP einer moi von 1 pfu/Zelle für jedes chimäre Virus infiziert. 48 Stunden nach der Infektion wurden die infizierten Zellen geerntet und in 1 ml SDS-Probenpuffer resuspendiert. Die Lysate wurden gründlich beschallt und weiter mit SDS-Probenpuffer verdünnt. 20 µl des verdünnten Lysats wurden auf einem 12 % SDS-Polyacrylamidgel aufgetrennt. Die Gele wurden entweder für den Transfer der Proteine auf einen Nitrocellulosefilter (Western Blot) verwendet, oder die Proteine wurden mit Coomassie-Blau angefärbt. Fig. 11A zeigt die immunologisch angefärbten Filter. Die Filter wurden mit einem Densitometer analysiert (Hirschmann Elscript 400). Die mit Coomassie-Blau angefärbten 1:30 Verdünnungen der Lysate aus jeder Infektion sind in Fig. 11B gezeigt. Die Ergebnisse der densitometrischen Analyse der Western Blot-Signale, einer geeigneten Verdünnung (1:10 für das vS1#10-Lysat; 1:50 für das vT7S1dM-Lysats), sind in Fig. 12 angegeben. Die beiden Peaks entsprechen den beiden preS1-Glycoformen. Betrachtet man die unterschiedlichen Verdünnungsfaktoren (1:10 gegenüber 1:50) und die Peakflächen (43 198 gegenüber 104 659), so ist klar, daß die Expression des modifizierten preS1-Gens ausgehend vom Bakteriophagen T7-Promotor 12 mal so stark ist wie die Expression des Wildtyp-preS1-Gens, ausgehend vom Vaccinia selP-Promotor.

### Beispiel 5 - Verstärkung der Proteinimmunogenizität: Konstruktion von Hybridproteinen, die aus dem HBV-Oberflächenglycoprotein und dem Diphtherie-Toxin Fragment A-kreuzreagierenden Material bestehen, und Verdopplung der HBV preS1-spezifischen Epitope

In einigen Fällen mag es wünschenswert sein, die Immunogenizität eines Hepatitis B-Virus-Impfstoffs durch Kopplung von HBV preS1-Peptiden an leistungsfähige Immunopotentiatoren zu verstärken. In diesem Beispiel wird ein neuer Immunopotentiator, nämlich Diphtherie-Toxin-kreuzreagierendes Material (CRM), eingesetzt, um die Immunantwort gegenüber HBV-Oberflächenantigenen zu verstärken. CRMs sind nicht funktionelle Formen des Diphtherie-Toxins, die mit Diphtherie-Toxin-Antiserum kreuzreagieren. Eine starke Immunantwort wird durch Corynebacterium diphtheriae, das CRM exprimiert, hervorgerufen; "Pharmacology of Bacterial Toxins", in: International Encyclopedia of Pharmacology and Therapeutics, Dorner und Drews (Herausgeber), S. 693-708 (1986). Das Diphtherie-Toxin-Gen oder CRM-Gen wird von einem lysogenen β-Phagen getragen, der in das bakterielle Genom integriert oder nicht integriert vorliegt. Das intakte Toxin besteht aus einem Fragment "A" mit einem Molekulargewicht von 22 kD und einem Fragment "B" mit einem Molekulargewicht von 40 kD. Das Fragment A sorgt für die toxischen enzymatischen Eigenschaften, während das Fragment B für die Translokation des Fragments A über die Zellmembran erforderlich ist. CRM 228 ähnelt dem Wildtyp-Toxin, weist jedoch keine enzymatische Aktivität aufgrund von Änderungen in der Aminosäuresequenz auf; Uchida et al., J. Biol. Chem., Bd. 248 (1973a), S. 3838-3844; Streeck et al., Science, Bd. 221 (1983), S. 855-858. Der Phage, der das Gen für CRM 228 trägt, ist vom Institut Pasteur, Paris, Frankreich, erhältlich.

Um die Immunogenität des demyristylierten großen Proteins (preS1-S2-S-sAg) oder der beiden kleineren HBV-Oberflächenglycoproteine preS2-S-sAg und S-sAg zu erhöhen, wird CRM 228 A mit diesen Antigenen fusioniert. Die Verknüpfung der für das Glykoprotein kodierenden DNA mit der Nucleinsäure, die für das CRM 228-Fragment A codiert, wird durch rekombinante DNA-Methoden erreicht; vgl. Fig. 13. Das Antigen und der Immunopotentiator sind also genetisch miteinander verbunden und werden als Fusionsprotein exprimiert. Das Fragment A wurde ausgewählt, da es im Vergleich zum CRM 228-Fragment B kleiner ist. Das CRM 228-Fragment B oder das vollständige Protein CRM 228 überschreiten die Größe des preS1-S2-S-sAg, was möglicherweise einen negativen Einfluß auf die Immunantwort, die gegen den preS1-Teil gerichtet ist, hat. Die geringere Größe des CRM 228-Fragments A ist vorteilhaft, indem sie die Zugänglichkeit der preS1-Epitope innerhalb des Fusionsproteins sicherstellt. Die starke Immunogenizität des Fragments A ist jedoch äquivalent zu der des nativen Moleküls.

Ein alternatives Verfahren zur Verknüpfung von HBV-Antigenen und Immunopotentiatoren ist die chemische Verknüpfung des hochgradig gereinigten CRM 228-Fragments A mit gereinigtem preS1-preS2-S-sAg (demyristylierte Version), preS2-S-sAg oder S-sAg. Zu diesem Zweck werden äquimolare Mengen an CRM 228-Fragment A und Antigen, die miteinander verbunden werden sollen, unter Verwendung von chemischen Standardverfahren vernetzt; z.B. Widder und Green, Methods in Enzymology, Bd. 112 (1985), S. 207.

Ein weiteres Verfahren zur Erhöhung der Immunogenizität von preS1 enthaltenden Antigenen besteht in der Verdopplung der preS1-Region des großen HBV-Antigens (Aminosäuren 1-108). Dies wird durch Insertion einer zweiten Kopie der für preS1 kodierenden Region in einen Vektor, der für das gesamte große HBV-Antigen codiert, erreicht; vgl. Fig. 14. Die Expression dieses Konstrukts ergibt das neue Protein (preS1)₂-preS2-S-sAg, das zwei B-und T-Zell-spezifische preS1-Epitope enthält.

### Beispiel 6 - Impfstoffe

Gemäß einer weiteren Ausführungsform werden die proteinhaltigen Partikel der vorliegenden Erfindung in einer pharmazeutisch verträglichen Zusammensetzung verwendet, die bei Verabreichung in einer wirksamen Menge imstande ist, eine schützende Immunität gegen eine HBV-Infektion zu induzieren.

Die Herstellung der Impfstoffe, die Proteine als Wirkstoffe enthalten, ist dem Fachmann bekannt. Typischerweise werden derartige Impfstoffe als injizierbare Impfstoffe, und zwar als flüssige Lösungen oder Suspensionen, hergestellt; feste Formen, die sich zum Lösen oder Suspendieren in einer Flüssigkeit vor der Injektion eignen, können ebenfalls hergestellt werden. Die Zubereitung kann auch emulgiert sein. Der aktive immunogene Bestandteil wird oftmals mit einem Träger gemischt, der pharmazeutisch verträglich und mit dem aktiven Bestandteil kompatibel ist. Geeignete Träger sind z.B. Wasser, Kochsalzlösung, Dextrose, Glycerin, Ethanol und dergl. sowie Kombinationen davon. Darüber hinaus kann der Impfstoff gegebenenfalls geringe Mengen an Hilfssubstanzen, wie Netzmitteln oder Emulgiermitteln, den pH-Wert puffernden Mitteln, Adjuvantien oder Immunopotentiatoren, die die Wirksamkeit des Impfstoffs erhöhen, enthalten.

Die Impfstoffe werden in herkömmlicher Weise parenteral durch Injektion, z.B. subkutan oder intramuskulär, verabreicht. Weitere Formulierungen, die für andere Arten der Verabreichung geeignet sind, umfassen Zäpfchen und in einigen Fällen orale Formulierungen. Für Zäpfchen können herkömmliche Bindemittel und Träger z.B. Polyalkalenglucose oder Triglyceride umfassen; Zäpfchen können aus Mischungen gebildet werden, die den Wirkstoff in einer Konzentration im Bereich von 0,5 % bis 10 % und vorzugsweise in einer Konzentration von 1,2 % enthalten. Orale Formulierungen umfassen typische Träger, wie Mannit, Lactose, Stärke, Magnesiumstearat, Natriumsaccharin, Cellulose, Magnesiumcarbonat und dergl., in pharmazeutischer Qualität. Die Zusammensetzungen nehmen die Form von Lösungen, Suspension, Tabletten, Pillen, Kapseln, Formulierungen für die verzögerte Freisetzung oder Pulvern an und enthalten 10 % bis 95 % und vorzugsweise 25 bis 70 % Wirkstoff.

Die proteinhaltigen Partikel können zu einem Impfstoff als neutrale Formen oder Salzformen formuliert werden. Pharmazeutisch verträgliche Salze umfassen Säureadditionssalze, gebildet mit den freien Aminogruppen des Peptids und anorganischen Säuren, z.B. Salzsäure oder Phosphorsäure, oder organischen Säuren, wie Essigsäure, Oxalsäure, Weinsäure, Mandelsäure oder dergl. Mit den freien Carboxylgruppen gebildete Salze können auch von anorganischen Basen, wie Natriumbydroxid, Kaliumhydroxid, Ammoniumhydroxid, Calciumhydroxid oder Eisen(III)-hydroxid, und organischen Basen, wie Isopropylamin, Trimethylamin, 2-Ethylaminoethanol, Histidin, Procain und dergl., abgeleitet werden.

Der Ausdruck "Einheitsdosis" bezieht sich auf physikalisch diskrete Einheiten, die sich für die Verwendung bei Menschen eignen, wobei jede Einheit eine vorbestimmte Menge an aktivem Material enthält, die so berechnet wurde, daß sie die gewünschte therapeutische Wirkung in Verbindung mit dem erforderlichen Verdünnungsmittel, d.h. dem Träger, und einem speziellen Behandlungsplan hervorruft. Die zu verabreichende Menge sowohl hinsichtlich der Anzahl der Behandlungen als auch der Menge hängt von dem zu behandelnden Patienten, der Fähigkeit des Immunsystems des Patienten, Antikörper zu synthetisieren, und dem gewünschten Grad des Schutzes ab. Genaue Mengen an Wirkstoff, die verabreicht werden müssen, hängen von der Beurteilung des Praktikers ab und gelten speziell für ein Individuum. Der geeignete Dosierungsbereich liegt jedoch in der Größenordnung von 1 bis mehreren 100 µg Wirkstoff pro Individuum. Geeignete Pläne für die anfängliche Verabreichung und Auffrischungen variieren ebenfalls, typischerweise folgen jedoch einer anfänglichen Verabreichung in Abständen von 1 oder 2 Wochen eine oder mehrere nachfolgende Injektionen oder andere Verabreichungen.

### Beispiel 7 - Herstellung monoclonaler Antikörper, die spezifisch für HBV ayw preS1 sind

3 Monate alte weibliche BALB/c-Mäuse wurden fünfmal mit gereinigtem HBV ayw preS1, das in Vero-Zellen, die mit der geeigneten Vaccinia-Virus-Chimäre infiziert worden waren, exprimiert wurde, in einer Impfversuchsreihe in einer Zeitspanne von 6 Wochen geimpft (ungefähr 1 bis 10 µg Protein pro Impfung). 3 Tage nach der letzten Impfung wurden den immunisierten Mäusen Blutproben entnommen und auf preS-spezifische Antikörper unter Verwendung eines ELISA-Systems mit preS1 als Testantigen untersucht. Milzzellen aus Mäusen mit einem positiven ELISA-Signal wurden isoliert und mit der Plasmacytom-Zellinie P3X63Ag8 (ATCC TIB 9) unter Verwendung von Polyethylenglykol gemäß dem Verfahren von Lane et al., J. Immunol. Meth., Bd. 81 (1985), S. 223-228, fusioniert. Hybridomzellklone, die preS-spezifische monoclonale Antikörper sezernieren, wurden in einem Verdünnungsassay in RPMI 1640-Medium (8041-2400, GIBCO) selektiert. Die Spezifität für den preS1-Teil der monoclonalen Antikörper wurde durch synthetische preS1-Peptide in einem handelsüblichen Peptid-Kartierungsreagenzsatz (Cambridge Research Biochemicals) bestätigt.

### Beispiel 8 - Diagnostische Systeme

Ein diagnostisches System, vorzugsweise in Form eines Reagenzsatzes, das sich für den Nachweis des Vorhandenseins von preS1 enthaltenden Antigenen oder Antikörpern gegen preS1 enthaltende Antigene in einer Körperprobe eignet, umfaßt (a) ein erfindungsgemäßes Protein oder (b) einen erfindungsgemäßen Antikörper und (c) ein markiertes spezifisches Bindungsmittel, um ein Signal beim Vorhandensein der Immunreaktion eines preS1 enthaltenden Proteins und eines anti-preS1 Antikörpers auszulösen.

Der Ausdruck "Markierung" in seinen verschiedenen grammatikalischen Formen bezieht sich auf einzelne Atome und Moleküle, die entweder direkt oder indirekt an der Auslösung eines nachweisbaren Signals beteiligt sind, um das Vorhandensein eines Immunreaktanten anzuzeigen. Beliebige Markierungsmittel können an ein spezifisches Agens gebunden oder diesem einverleibt oder getrennt verwendet werden, und diese Atome oder Moleküle können allein oder zusammen mit weiteren Reagenzien verwendet werden. Derartige Markierungen sind als solche im Bereich der Immunchemie bekannt und stellen einen Teil der vorliegenden Erfindung nur insofern dar, als sie mit in anderer Hinsicht neuen Proteinen, Verfahren und/oder Systemen verwendet werden.

Die erfindungsgemäßen diagnostischen Reagenzsätze werden typischerweise in einem "ELISA-Format" verwendet, um das Vorhandensein oder die Menge von anti-preS1-Antikörpern oder preS1 enthaltenden Proteinen in einer Körperprobe, wie Serum oder Plasma, nachzuweisen. "ELISA" bezieht sich auch auf einen Festphasen-Enzym-Immunoassay ("enzyme-linked immunosorbent assay"), bei dem ein Antikörper oder Antigen, gebunden an eine feste Phase, und ein Enzym-Antigen- oder Enzym-Antikörper-Konjugat verwendet werden, um die Menge an Antigen oder Antikörper, die in einer Probe vorhanden ist, nachzuweisen und quantitativ zu bestimmen. Eine Beschreibung der ELISA-Technik findet sich in Kapitel 22 der 4. Auflage von Basic and Clinical Immunology, D.P. Sites et al., Lange Medical Publications, Los Altos, CA (1982) und in den US-Patenten 3 654 090, 3 850 752 und 4 016 043, wobei alle diese Druckschriften durch entsprechenden Verweis zum Gegenstand der vorliegenden Anmeldung gemacht werden.

Gemäß bevorzugter Ausführungsformen sind das erfindungsgemäße preS1 enthaltende Protein oder der erfindungsgemäße anti-preS1-Antikörper an einer festen Matrix fixiert, wobei ein fester Träger gebildet wird. Die Moleküle werden an der festen Matrix typischerweise durch Adsorption aus einem wäßrigen Medium fixiert, wobei jedoch auch mehrere andere Arten der Adsorption sowie andere Arten der Fixierung, die dem Fachmann bekannt sind, herangezogen werden können. Beispielhaft für derartige Arten sind die Wechselwirkung von Proteinen mit den Carboxyleinheiten, die durch Reaktion von Bromcyan mit Glucose enthaltenden Matrices gebildet werden. Diese Matrices umfassen vernetzte Dextrose oder Cellulose und eine Verknüpfung mit Glutaraldehyd, wie es nachstehend im Zusammenhang mit Latexteilchen und dergl. erläutert wird.

Geeignete feste Matrices sind dem Fachmann bekannt. Derartige Materialien umfassen das vernetzte Dextran, das unter der Warenbezeichnung SEPHADEX von Pharmacia Fine Chemicals (Piscataway, NJ), erhältlich ist, Agarose, Kügelchen aus Polystyrol von etwa 1 µm bis etwa 5 mm Durchmesser (Abbott Laboratories , North Chicago, IL), Polyvinylchlorid, Polystyrol, vernetztes Polyacrylamid, auf Nitrocellulose oder auf Nylon basierende Gewebe, wie Lagen, Streifen, Schaufeln und Rohre. Eine bevorzugte Matrix weist die Form einer Platte oder einer Vertiefung einer Mikrotiterplatte auf, wie diejenigen, die aus Polystyrol oder Polyvinylchlorid gefertigt sind. Eine Mikrotiterplatte und ein oder mehrere Puffer können ebenfalls als Bestandteile dem diagnostischen Reagenzsatz einverleibt werden.

### Literaturverzeichnis:

Bancroft, W.H., Mundon, F.K., Russell, P.K. 1972. Detection of additional antigenic determinants of hepatitis B antigen. J. Immunol. 109, 842.
Belyacv, A.S., Roy, P. 1992. Presentation of Hepatitis B. Virus preS2 Epitope on Bluetongue Virus Core-like Particles. Virology 190, 840-844.
Cheng, K.C., Moss, B. 1987. Selective Synthesis and Secretion of Particles Composed of the Hepatitis B. Virus Middle Surface Protein Directed by a Recombinant Vaccina Virus: Induction of Antibodies to Pre-S and S Epitopes. J. Virol., 61, 1286-1290.
Cheng, K.C., Smith, G.L., Moss, B. 1986. Hepatitis B Virus Large Surface Protein Is not Secreted but Is Immunogenic when Selectively Expressed by Recombinant Vaccinia Virus. J. Virol., 60, 337-344.
Christman, J.K., Gerber, M., Price, P.M., Flordellis, C., Edelman, J., Ace, G. 1982. Amplification of expression of hepatitis B surface antigen in 3T3 cells cotransfected with a dominant acting gene and cloned viral DNA. Proc. Natl. Acad. Sci. USA 79, 1815-1819.
Dehoux, P., Ribes, V., Sobczak, E., Streeck, R.E. 1986. Expression of the hepatitis B virus large envelope protein in Saccharmoyces cerevisiae. Gene. 48, 155-163.
Egea et al., 1991. The cellular basis for lack of antibody response to hepatitis B vaccine in humans. J. Exp. Med. 173, 531-538.
Elroy-Stein, O., Fuerst, T.R., Moss, B. 1989. Cap-independent translation of mRNA conferred by encephalomyocarditis virus 5' sequence improves the performance of the vaccinia virus/bacteriophage T7 hybrid expression system. Proc. Natl. Acad. Sci. USA 86,, 6126-6130.
Fuerst, T.R., Niles, E.G., Studier, W.F., Moss, B. 1986. Eukaryotic transient expression system based on recombinant vaccinia virus that synthesizes bacteriophage T7 RNA polymerase. Proc. Natl. Acad. Sci. USA 83: 8122-8126.
Ganem, D. and Varmus, H.E. 1987. The molecular biology of Hepatitis B viruses. Ann. Rev. Biochem. 56, 561-693.
Hollinger, F.B. 1990. Hepatitis B virus. In Virology eds. Fields, B.N., Knipe D.M. Raven Press, N.Y., 2171-2236.
Imamura, T., Araki, M., Miyanohara, A., Nakao, J., Yonemura, H., Ohtomo, N., Matsubara, K. 1987. Expression of Hepatitis B Virus Middle and Large Surface Antigen Genes in Saccharomyces cerevisiae. J. Virol. 61, 3543-3549.
Korec, E., Gerlich, W.H. 1992. Expression of large hepatitis B envelope protein mutants using a new expression vector. Arch. Virol., 122, 367-371.
Kumar, V., Bansal, V.J., Rao, K.V.S., Jameel, S. 1992. Hepatitis B virus envelope epitopes: gene assembly and expression in Escherichia coli of an immunologically reactive novel multiple-epitope polypeptide 1 (MEP-1). Gene. 110, 137-144.
Kuroda, S., Fujisawa, Y., Iino, S., Akahane, Y., Suzuki, H. 1992a. Induction of protection level of anti-pre-S2 antibodies in humans immunized with a novel hepaptitis B vaccine consisting of M (pre-S2+S) protein particles (a third generation vaccine). Vaccine, 9, 163.
Kutinova, L., Nemeckova, S., Hamsikova, E., Press, M., Zavadova, H., Hirsch, L., Nemeeck, V., Smrt, J., Slonim, D., Vonka, V. 1990. A recombinant vaccina virus expressing hepatitis B virus middle surface protein. Restricted expression of HBV antigens in human diploid cells. Arch. Virol., 112, 181-193.
Le Bouvier, G.L. 1971. The heterogeneity of the Australia antigen. J. Infect. Dis. 123, 671.
Lee, T., Inokoshi, J., Namiki, M., Takeshima, H., Omura, S. 1989. Production of hepatitis B virus surface antigen containing pre-S1 and pre-S2 domains by Chinese hamster ovary cells. Arch. Virol., 106, 151-158.
Lin, Y., Liu, Y.X., Cislo, T., Mason, B.L., Yu, M.Y.W. 1991. Expression and Characterization of the PreS1 Peptide of Hepatitis B Surface Antigen in Escherichia coli. Journal of Medical Virology, 33, 181-187.
Masuda, M., Yuasa, T., Yoshikura, H. 1990. Effect of the PreS1 RNA Sequence on the Efficiency of the Hepatitis B Virus PreS2 and S Protein Translation. Virology, 174, 320-324.
Mayr, A. and Malicki, K. (1966). Attenuierung von virulentem Hühnerpockenvirus in Zellkulturen und Eigenschaften des attentuierten Virus. Zentralblatt f. Veterinärmedizin. Reihe B, 13; 1-12.
McLachlan, A., Milich, D.R., Raney, A.K., Riggs, M.G., Hughes, J.L., Sorge, J., Chisari, F.V. 1987. Expression of Hepatitis B Virus Surface and core Antigens: Influences of Pre-S and Precore Sequences. J. Virol., 61, 683-692.
Milich, D.R., Mc Namara, M.K., Mc Lachlan, A., Thornthon, G.B., Chisari, F.V. 1985a. Distinct H-2-linked regulation of T-cell responses to the pre-S and S regions of the same hepatitis B surface antigen polypeptide allows circumvention of nonresponsiveness to the S region. Proc. Natl. Acad. Sci. USA 82, 8168-8172.
Milich, D.R., Thornton, G.B., Kent, S.B., Michel, M.L., Tiollais, P. 1985b. Enhanced Immunogenicity of the Pre-S Region of Hepatitis B Surface Antigen. Science, 228, 1195-1199.
Milich et al., 1990a. Importance of subtype in the immune response to the pre-S2 region of the hepatitis B surface antigen. I. T cell fine specificity. J. Immunol. 144, 3535-3543.
Milich et al., 1990b. Importance of subtype in the immune response to the pre-S2 region of the hepatitis B surface antigen. II. Synthetic preS2 immunogen. J. Immunol. 144, 3544-3551.
Moss, B., Elroy-Stein, O., Mizukami T., Alexander, W.A., Fuerst, T.R. Product review: new mammalian expression vectors. Nature 348, 91-92 (1990).
Nemeckova, S., Kutinova, L., Hamsikova, E., Kunke, D., Press, M., Zvadova, H., Smrt, J., Vonka, V. 1991. Synthesis and immunogenicity of hepatitis B virus envelope antigen expressed by recombinant vaccinia virus. Finding of retention signal in the C-terminal portion of the preS1 domain of subtype adyw. Arch. Virol., 121, 29-41.
Neurath, A.R., Kent, S.B.H., Strick, N. 1990. Location and Chemical Synthesis of Pre-S Gene Coded Immunodominant Epitope of Hepatitis B Virus. Science 224, 392-394.
Neurath, A.R., Kent, S.B.H., Strick, N., Taylor, P., Stevens, C.E. 1985. Hepatitis B virus contains pre-S gene-encoded domains. Nature 315,, 154-156.
Neurath, A.R., Seto, B., Strick, N. 1989b. Antibodies to synthetic peptides from the preS1 region of the hepatitis B virus (HBV) envelope (env) protein are virus-neutralizing and protective. Vaccine 7, 234-236.
Nishioka K., Levin, A.G., Simons, M.J. 1975. Hepatitis B. antigen, antigen subtypes, and hepatitis B antibody in normal subjects and patients with liver disease. Bulletin of the WHO 52, 293-300.
Ou, J.-H. Rutter, W.J. 1987. Regulatin of Secretion of the Hepatitis B Virus Major Surface Antigen by the PreS-1 Protein. Journal of Virology 61, 782-786.
Persing, D.H. Varmus, H.E., Ganem, D. 1987. The preS1 protein of hepatitis B virus is acylated at its amino terminus with myristic acid. J. Virol. 61, 1672-1677.
Petit, M.A., Capel, F., Dubanchet, S., Mabit, H. 1992. PreS1-Specific Binding Proteins as Potential Receptors for Hepatitis B Virus in Human Hepatocytes. Virology, 187, 211-222.
Pontisso, P., Petit, M.A., Bankowski, M.J., Peeples, M.E. 1989a. Human Liver Plasma Membranes Contain Receptors for the Hepatitis B Virus Pre-S1 Region and, via Polymerized Human Serum Albumin, for the Pre-S2 Region. Journal of Virology, 63, 1981-1988.
Pontisso, P., Ruvoletto, M.G., Gerlich, W.H., Heermann, K.H., Bardini, R., Alberti, A. 1989b. Identification of an Attachment Site for Human Liver Plasma Membranes on Hepatitis B Virus Particles. Virology 173, 522-530.
Prange, R., Nagel, R., Streek, R. 1992. Deletions in the Hepatitis B Virus Small Envelope Protein: Effect on Assembly and Secretion of Surface Antigen Particles. Journal of Virology, 66, 5832-5841.
Robinson, W.S. 1990. Hepadnaviridac and their replication. In *Virology* eds. Fields B.N, Knipe D.M. pp. 2137-2169. Raven Press, New York.
Scheiflinger, F., Dorner, F., Falkner, F.G. 1992. Construction of chimeric vaccinia virus by molecular cloning and packaging. Proc. Natl. Acad. Sci. USA 89, 9977-9981.
Schödel, F., Will, H., Johansson, S., Sanchez, J., Holmgren, J. 1991. Synthesis in Vibrio cholerae and secretion of hepatitis B virus antigens fused to Escherichia coli heat-labile enterotoxin subunit B. Gene, Science, 228, 255-259.
Shiosaki, K. Takata, K.I., Nishimura, S.I., Mizokami, H., Mitsubara, K. 1991. Production of hepatitis B virion-like particles in yeast. Gene, 106, 143-149.
Shiraki, K., Hayakawa, Y., Mori, H., Namazue, J., Takamizawa, A., Yoshida, I., Yamanishi, K., Takahashi, M. 1991. Development of immunogenic recombinant Oka varicella vaccine expressing hepatitis B virus surface antigen. Journal of General Virology, 72, 1393-1399.
Szmuness, W., Stevens, C.E., Harley, E.J., et al., 1982. Hepatitis B vaccine in medical staff of hemodialysis units. N. Engl. J. Med., 3077, 1481-1486.
Szmuness, W., Stevens, C.E., Zang, E.A., Harley, E.J., Kellner, A.A. 1980. New England J. Med., 303, 833-841.
Towbin, H., Stachclin, T., Gordon, J. (1979). Electrophorctic transfer of proteins from polyacrylamid gels to nitrocellulose sheets: procedure and some applications. Proc. Natl. Acad. Sci. USA 76, 4350-4354.
Venkatesan, S., Baroudy, B.M., Moss, B. (1981). Distinctive nucleotide sequences adjacent to multiple initiation and termination sites of an early vaccinia virus gene. Cell 25, 805-813.
Youn, B.W., Samanta, H. 1989. Purification and characterization of pre-S-containing hepatitis B surface antigens produced in recombinant mammalian cell culture. Vaccine, 7, 60-68.
Yu, M.W., Shih, J.W.-K-., Bursztyn-Pettegrew, H., Byars, N.F., Allison, A.C., Chan, H.W. 1991. Expression of Pre-S2 Region of Hepatitis B Surface Antigen in Escherichia coli. Journal of Medical Virology 30, 7-13.
Yuasa, T., Kajino, K., Saito, I., Miyamura, T. 1991. Preferential expression of the large hepatitis B virus surface antigen gene by an adenovirus-hepatitis B virus recombinant. Journal of General Virology, 72, 1972-1984.

### Patent Literatur

EP 0 198 474 A1, Salstroem et al. Hepatitis B surface antigen formed by recombinant DNA techniques, vaccines, diagnostics cell lines and methods forming same. ENDOTRONICS INC.
(preS1-S2-S protein containing particles produced in mammalian cells (CHO, Vero, mouse L Cells))
EP 0 414 374 A2. Comberbach et al. 1990. Novel antigens and methods for their preparation. SMITHKLINE BIOLOGICALS.
(preS1-S2-S protein containing particles produced in Hansenula yeasts)
U.S. Serial No. 07/750,080. Dorner et al. 1991. Patent application "Direct molecular cloning of a modified eukaryotic cytoplasmic DNA virus genome".
U.S. Serial No. 07/750,080 CIP. Dorner et al. 1992. Continuation in Part of "Direct molecular cloning of a modified eukaryotic cytoplasmic DNA virus genome".
U.S. 5,077,213. Li et al. Recombinant vaccinia virus. SHANGHAI INSTITUTE OF BIOCHEMISTRY.
(preS1-S2-S protein containing particles produced in vaccinia)
PCT WO 85/03876. Sobczak et al., 1985. Composition useful for. . . INSTITUT PASTEUR.
(preS2-S protein containing particles produced in CHO)
EP 0 174 444 B1. Valenzuela, P.T. 1984. Hepatitis surface antigen particle vaccine. CHIRON CORPORATION
(preS2-S protein containing particles produced in yeast)
EP A 0 312 159. Ellis et al., 1987. Method for producing hepatitis B proteins in yeast. MERCK & CO.
(preS2-S protein containing particles produced in Saccharomyces)
PCT WO 90/10058. Even-Chen, 1989. Method for production and purification of hepatitis B vaccine. BIO-TECHNOLOGY GENERAL CORP.
(preS1-S2-S containing particles produced in CHO cells)
US Serial# 07/882,768. Falkner et al., 1992. Patent application "Production of isolated proteinaceous materials using recombinant avipox virus vectors".
EP A 91 114 300.-6. Dorner et al., 1991. European patent application "Recombinant Fowlpox Virus (Intergenic region)".

## Patentansprüche

1. Chimäres Vaccinia-Virus, umfassend einen Promotor in funktioneller Verknüpfung mit einem Polynucleotid, das für einen Bereich eines großen Antigens eines Hepatitis B-Virus codiert, der mindestens ein preS1-B- oder -T-Zell-Epitop aufweist, wobei dem Bereich die Myristylierungsstelle der preS1-Region fehlt.

2. Virus nach Anspruch 1, ferner umfassend einen selektierbaren Marker.

3. Virus nach Anspruch 2, wobei es sich bei dem Marker um das E. coli-gpt-Gen handelt.

4. Virus nach Anspruch 1, wobei es sich bei dem Promotor um einen Bacteriophage T7-Promotor handelt.

5. Virus nach Anspruch 1, wobei es sich bei dem Promotor um einen frühen/späten Vaccinia-Promotor handelt.

6. Virus nach Anspruch 1, wobei das Polynucleotid für mindestens ein B-Zell-Epitop und mindestens ein T-Zell-Epitop codiert.

7. Virus nach Anspruch 6, wobei das Polynucleotid für mehr als ein B- oder T-Zell-Epitop codiert.

8. Virus nach Anspruch 7, wobei das Polynucleotid die gesamte für preS1 codierende Region enthält.

9. Virus nach Anspruch 8, wobei das Polynucleotid die gesamte codierende Region des großen Antigens enthält.

10. Virus nach Anspruch 8, wobei das Polynucleotid mindestens zwei Kopien der gesamten für preS1 codierenden Region enthält.

11. Virus nach Anspruch 8, wobei das Polynucleotid die codierende Sequenz für einen Proteinimmunpotentiator enthält.

12. Virus nach Anspruch 11, wobei es sich bei dem Immunpotentiator um das CRM 228-Fragment A handelt.

13. Verfahren zur Synthese eines Hepatitis B-Virus-Antigens, das folgende Stufen umfaßt:
(a) Infektion einer geeigneten eukaryontischen Zelle mit
(i) einem ersten chimären Vaccinia-Virus, das einen Promotor in funktioneller Verknüpfung mit einem Polynucleotid umfaßt, das für einen Bereich der preS1-Region des großen Hepatitis B-Virus-Antigens codiert, der mindestens ein preS1-B- oder -T-Zell-Epitop enthält, wobei dem Bereich die Myristylierungsstelle der preS1-Region fehlt;
(b) Kultivieren der coinfizierten Zelle;
(c) Lyse der coinfizierten Zelle; und
(d) Gewinnung des Antigens.

14. Verfahren nach Anspruch 13, das ferner die Stufe der Reinigung des Antigens umfaßt.

15. Verfahren nach Anspruch 13, wobei es sich bei dem Promotor um einen frühen/späten Vaccinia-Promotor handelt.

16. Verfahren nach Anspruch 13, wobei es sich bei dem Promotor um einen Bakteriophagen T7-Promotor handelt, wobei die geeignete Zelle mit einem zweiten chimären Vaccinia-Virus coinfiziert wird, das ein Polynucleotid umfaßt, das für eine Bakteriophagen T7-RNA-Polymerase codiert.

17. Verfahren nach Anspruch 13, wobei die eukaryontische Zelle aus der Gruppe der Säugernierenzellen oder der Säugerleberzelle ausgewählt ist.

18. Verfahren nach Anspruch 13, wobei das Polynucleotid die gesamte für preS1 codierende Region enthält.

19. Verfahren nach Anspruch 18, wobei das Polynucleotid die gesamte codierende Region für das große Antigen enthält.

20. Verfahren nach Anspruch 18, wobei das Polynucleotid mindestens zwei Kopien der gesamten für preS1 codierenden Region enthält.

21. Verfahren nach Anspruch 18, wobei das Polynucleotid die codierende Sequenz für einen Proteinimmunpotentiator enthält.

22. Verfahren nach Anspruch 21, wobei es sich bei dem Immunpotentiator um das CRM 228-Fragment A handelt.

23. Hepatitis B-Virus-Antigen, hergestellt nach einem Verfahren, das folgende Stufen umfaßt:
(a) Coinfektion einer geeigneten eukaryontischen Zelle mit
(i) einem ersten chimären Vaccinia-Virus, das einen Promotor in funktioneller Verknüpfung mit einem Polynucleotid umfaßt, das für einen Bereich der preS1-Proteinregion eines großen Hepatitis B-Virus-Antigens codiert, der mindestens ein preS1-B- oder -T-Zell-Epitop enthält, wobei dem Bereich die Myristylierungsstelle der preS1-Region fehlt;
(b) Kultivieren der coinfizierten Zelle;
(c) Lyse der coinfizierten Zelle; und
(d) Gewinnung des Antigens.

24. Antigen nach Anspruch 23, hergestellt nach der weiteren Stufe der Reinigung des Antigens.

25. Antigen nach Anspruch 23, wobei es sich bei dem Promotor um einen frühen/späten Vaccinia-Promotor handelt.

26. Antigen nach Anspruch 23, wobei es sich bei dem Promotor um einen Bakteriophagen T7-Promotor handelt, wobei die geeignete Zelle mit einem zweiten chimären Vaccinia-Virus coinfiziert wird, das ein Polynucleotid umfaßt, das für eine Bakteriophagen T7-RNA-Polymerase codiert.

27. Antigen nach Anspruch 23, wobei die eukaryontische Zelle aus der Gruppe der Säugernierenzelle oder der Säugerleberzelle ausgewählt ist.

28. Antigen nach Anspruch 22, wobei das Polynucleotid die gesamte für preS1 codierende Region enthält.

29. Antigen nach Anspruch 23, wobei das Polynucleotid die gesamte codierende Region für das große Antigen enthält.

30. Antigen nach Anspruch 23, wobei das Polynucleotid mindestens zwei Kopien der gesamten für preS1 codierenden Region enthält.

31. Antigen nach Anspruch 23, wobei das Polynucleotid die codierende Sequenz für einen Proteinimmunpotentiator enthält.

32. Antigen nach Anspruch 31, wobei es sich bei dem Immunpotentiator um das CRM 228-Fragment A handelt.

33. Impfstoff, umfassend ein Antigen des Hepatitis B-Virus, das gemäß einem Verfahren hergestellt wurde, das folgende Stufen umfaßt:
(a) Coinfektion einer geeigneten eukaryontischen Zelle mit
(i) einem ersten chimären Vaccinia-Virus, das einen Promotor in funktioneller Verknüpfung mit einem Polynucleotid umfaßt, das für einen Bereich der preS1-Proteinregion eines großen Hepatitis B-Virus-Antigens codiert, der mindestens ein preS1-B- oder -T-Zell-Epitop enthält, wobei dem Bereich die Myristylierungsstelle der preS1-Region fehlt;
(b) Kultivieren der coinfizierten Zelle;
(c) Lyse der coinfizierten Zelle; und
(d) Gewinnung des Antigens;
und einen pharmazeutisch verträglichen Träger.

34. Impfstoff nach Anspruch 33, hergestellt nach der weiteren Stufe der Reinigung des Antigens.

35. Impfstoff nach Anspruch 33, wobei es sich bei dem Promotor um einen frühen/späten Vaccinia-Promotor handelt.

36. Impfstoff nach Anspruch 33, wobei es sich bei dem Promotor um einen Bakteriophagen T7-Promotor handelt, wobei die geeignete Zelle mit einem zweiten chimären Vaccinia-Virus coinfiziert wird, das ein Polynucleotid umfaßt, das für eine Bakteriophagen T7-RNA-Polymerase codiert.

37. Impfstoff nach Anspruch 33, wobei die eukaryontische Zelle aus der Gruppe der Säugernierenzelle oder der Säugerleberzelle ausgewählt ist.

38. Impfstoff nach Anspruch 33, wobei das Polynucleotid die gesamte für preS1 codierende Region enthält.

39. Impfstoff nach Anspruch 33, wobei das Polynucleotid die gesamte codierende Region für das große Antigen enthält.

40. Impfstoff nach Anspruch 33, wobei das Polynucleotid mindestens zwei Kopien der gesamten für preS1 codierenden Region enthält.

41. Impfstoff nach Anspruch 33, wobei das Polynucleotid die codierende Sequenz für einen Proteinimmunpotentiator enthält.

42. Impfstoff nach Anspruch 41, wobei es sich bei dem Immunpotentiator um das CRM 228-Fragment A handelt.

43. Anti-HBV-preS1-Antikörper, hergestellt nach einem Verfahren, das folgende Stufen umfaßt:
(a) Coinfektion einer geeigneten eukaryontischen Zelle mit
(i) einem ersten chimären Vaccinia-Virus, das einen Promotor in funktioneller Verknüpfung mit einem Polynucleotid umfaßt, das für einen Bereich der preS1-Proteinregion eines großen Hepatitis B-Virus-Antigens codiert, der mindestens ein preS1-B- oder -T-Zell-Epitop enthält, wobei dem Bereich die Myristylierungsstelle der preS1-Region fehlt;
(b) Kultivieren der coinfizierten Zelle;
(c) Lyse der coinfizierten Zelle;
(d) Gewinnung des Antigens;
(e) Immunisierung eines Säugers mit dem Antigen; und
(f) Isolierung von Antikörpern, die spezifisch für das Antigen sind,
aus dem Säuger.

44. Antikörper nach Anspruch 43, hergestellt nach der weiteren Stufe der Reinigung des Antigens.

45. Antikörper nach Anspruch 43, wobei es sich bei dem Promotor um einen frühen/späten Vaccinia-Promotor handelt.

46. Antikörper nach Anspruch 43, wobei es sich bei dem Promotor um einen Bakteriophagen T7-Promotor handelt, wobei die geeignete Zelle mit einem zweiten chimären Vaccinia-Virus coinfiziert wird, das ein Polynucleotid umfaßt, das für eine Bakteriophagen T7-RNA-Polymerase codiert.

47. Antikörper nach Anspruch 43, wobei die eukaryontische Zelle aus der Gruppe der Säugernierenzelle oder der Säugerleberzelle ausgewählt ist.

48. Antikörper nach Anspruch 43, wobei das Polynucleotid die gesamte codierende Region für das große Antigen enthält.

49. Diagnostischer Reagenzsatz, umfassend ein Hepatitis B-Virus-Antigen, das nach einem Verfahren hergestellt wurde, das folgende Stufen umfaßt:
(a) Coinfektion einer geeigneten eukaryontischen Zelle mit
(i) einem ersten chimären Vaccinia-Virus, das einen Promotor in funktioneller Verknüpfung mit einem Polynucleotid umfaßt, das für einen Bereich der preS1-Proteinregion eines großen Hepatitis B-Virus-Antigens codiert, der mindestens ein preS1-B- oder -T-Zell-Epitop enthält, wobei dem Teil die Myristylierungsstelle der preS1-Region fehlt;
(b) Kultivieren der coinfizierten Zelle;
(c) Lyse der coinfizierten Zelle; und
(d) Gewinnung des Antigens;
und einen Behälter, der das Antigen enthält.

50. Reagenzsatz nach Anspruch 49, hergestellt nach der weiteren Stufe der Reinigung des Antigens.

51. Reagenzsatz nach Anspruch 49, wobei es sich bei dem Promotor um einen frühen/späten Vaccinia-Promotor handelt.

52. Reagenzsatz nach Anspruch 49, wobei es sich bei dem Promotor um einen Bacteriophage T7-Promotor handelt, wobei die geeignete Zelle mit einem zweiten chimären Vaccinia-Virus coinfiziert wird, das ein Polynucleotid umfaßt, das für eine Bakteriophagen T7-RNA-Polymerase codiert.

53. Reagenzsatz nach Anspruch 49, wobei die eukaryontische Zelle aus der Gruppe der Säugernierenzelle oder der Säugerleberzelle ausgewählt ist.

54. Reagenzsatz nach Anspruch 49, wobei das Polynucleotid die gesamte codierende Region für das große Antigen enthält.

55. Diagnostischer Reagenzsatz, umfassend einen anti-HBV-preS1-Antikörper, der nach einem Verfahren hergestellt wurde, das folgende Stufen umfaßt:
(a) Coinfektion einer geeigneten eukaryontischen Zelle mit
(i) einem ersten chimären Vaccinia-Virus, das einen Promotor in funktioneller Verknüpfung mit einem Polynucleotid umfaßt, das für einen Bereich der preS1-Proteinregion eines großen Hepatitis B-Virus-Antigens codiert, der mindestens ein preS1-B- oder -T-Zell-Epitop enthält, wobei dem Bereich die Myristylierungsstelle der preS1-Region fehlt;
(b) Kultivieren der coinfizierten Zelle;
(c) Lyse der coinfizierten Zelle;
(d) Gewinnung des Antigens;
(e) Immunisierung eines Säugers mit dem Antigen; und
(f) Isolierung von Antikörpern, die spezifisch für das Antigen sind,
aus dem Säuger;
und einen Behälter, der den Antikörper enthält.

56. Reagenzsatz nach Anspruch 55, hergestellt nach der weiteren Stufe der Reinigung des Antigens.

57. Reagenzsatz nach Anspruch 55, wobei es sich bei dem Promotor um einen frühen/späten Vaccinia-Promotor handelt.

58. Reagenzsatz nach Anspruch 55, wobei es sich bei dem Promotor um einen Bakteriophagen T7-Promotor handelt, wobei die geeignete Zelle mit einem zweiten chimären Vaccinia-Virus coinfiziert wird, das ein Polynucleotid umfaßt, das für eine Bakteriophagen T7-RNA-Polymerase codiert.

59. Reagenzsatz nach Anspruch 55, wobei die eukaryontische Zelle aus der Gruppe der Säugernierenzelle oder der Säugerleberzelle ausgewählt ist.

60. Reagenzsatz nach Anspruch 55, wobei das Polynucleotid die gesamte codierende Region für das große Antigen enthält.
